# EUROPEAN PATENT APPLICATION

(11) **EP 4 800 107 A1**
(43) Date of publication of application: **02.09.2026**
(21) Application number: 24881727.2
(22) Date of filing: 25.10.2024
(51) Int. Cl.: C12N 15/00, C12N 15/09, C12N 15/61, C12N 1/21, C12R 1/19

(54) **HOST CELL FOR IMPROVING STABILITY OF POLY(A) PLASMID**

(30) Priority: 25.10.2023 CN 202311399354
(71) Applicant: Nanjing GenScript Biotech Co., Ltd., Nanjing, Jiangsu 211100 (CN)
(72) Inventor: MENG, Qingwei, Nanjing, Jiangsu 211100 (CN); ZHANG, Menglei, Nanjing, Jiangsu 211100 (CN); ZUO, Wenlu, Nanjing, Jiangsu 211100 (CN); WEI, Kaiying, Nanjing, Jiangsu 211100 (CN); WANG, Shiqin, Nanjing, Jiangsu 211100 (CN)
(74) Representative: Synergy IP Group AG
(86) International application number: PCT/CN2024/127251
(87) International publication number: WO 2025/087366

(57) **Abstract**

Provided is the use of an *Escherichia coli* host cell in the replication or expression of a nucleic acid molecule containing a poly(A) sequence, wherein the *Escherichia coli* host cell comprises a down-regulated gyrase activity or a down-regulated gyrase expression level.

## Description

### Cross-Reference to Related Applications

The present application claims priority to Chinese patent application No. 202311399354.3, filed on October 25, 2023, which is incorporated herein by reference in its entirety.

### Technical Field

The present application relates to the use of an *Escherichia coli* host cell in the replication or expression of a nucleic acid molecule containing a poly(A) sequence, wherein the *Escherichia coli* host cell comprises a down-regulated gyrase activity or a down-regulated gyrase expression level. The stability of replication or expression of the poly(A) sequence may be enhanced using the *Escherichia coli* host cell to replicate or express a nucleic acid molecule containing the poly(A) sequence.

### Background

An mRNA vaccine is a type of nucleic acid formulation and is an mRNA prepared by transcription, synthesis and other processes from a sequence of an exogenous gene of interest. This mRNA may be introduced into body cells through a specific delivery system to express a protein of interest, thereby stimulating the body to produce a specific immunological response and enabling the body to obtain immune protection. Compared with traditional vaccines, the mRNA vaccine has some outstanding advantages, such as ease of design, rapid production speed, low cost, ability to induce cellular immunity and humoral immunity, and no interaction with genomic DNA. In recent years, various types of mRNA vaccines have achieved breakthrough progress in basic research and clinical research. In particular, the applications of mRNA vaccines in fields such as viral infectious diseases (e.g., novel coronavirus, influenza virus, HIV, and rabies virus), immuno-oncology (IO), personalized cancer vaccines (PCV), genetic diseases and rare diseases have shown a sustained growth trend.

The main sequence of a mature mRNA is the coding region, with untranslated regions (UTR) located on its upstream and downstream sides. Eukaryotic mRNA molecules also have a 5' cap at one end and a 3' poly(A) tail structure at the other end, wherein the poly(A) tail plays an important role in maintaining mRNA stability, regulating mRNA translation efficiency and maintaining mRNA transportation.

For large-scale synthesis of mRNA, the currently relatively efficient method is in vitro transcription (IVT). The IVT mainly involves the preparation of mRNA using linear DNA as the template. The main process steps include transcription of linearized plasmid DNA into mRNA, chemical modification (e.g., 5'-end capping, 3'-end poly(A) tailing), and separation and purification, and the like.

When mRNA is synthesized by IVT, there are two main methods for poly(A) tailing.

The first method is an enzymatic synthesis method, i.e. addition of poly(A) polymerase derived from *Escherichia coli* after the completion of mRNA transcription. The advantages of this method are no need for a template and simple operation, but it has the limitation of unstable tailing length.

The second method is a co-transcription method, in which poly(A) tail is formed by direct transcription of the poly(A) sequence already existing on a template plasmid DNA or PCR product. Its advantages lie in no need for poly(A) polymerase, reduction of process steps and cost saving, but it has the problem of easy deletion of the poly(A) sequence. Large-scale production of plasmids is usually performed through *Escherichia coli* fermentation. During the amplification process, plasmids carrying longer poly(A) sequences are unstable in themselves, and the coding sequences of the poly(A) tails are prone to deletion during the replication process, resulting in plasmid heterogeneity. In order to improve the stability of the poly(A) sequences on the plasmids, some scientists have used the segmented poly(A) sequence co-transcription method. Since each poly(A)-binding protein (PABP) only binds to about 30 As, a small amount of As act as a spacer sequence between the two binding proteins. By replacing the spacer sequence with other non-A bases, the probability of recombination of the poly(A) tail coding sequences is reduced. In addition, the length and bases of the spacer sequence may be optimized to further reduce the risk of poly(A) tail deletion. Nevertheless, the risk of A deletion still exists, and plasmids with poly(A) prepared via segmented method will have a certain impact on the expression level of downstream proteins. In addition, it has been reported that the integrity of the poly(A) tail coding sequence in plasmids may be improved by fermentation at a low temperature (30°C). However, bacterial growth slows down at a low temperature, the copy number of plasmids decreases, the output significantly reduces, and the cycle lengthens, which poses difficulties for plasmid production.

There are currently no good methods to avoid deletion of A bases during poly(A) plasmid construction and amplification in *Escherichia coli*. Some recombination-deficient commercial strains such as NEB stable and Stabl3 are often used for construction and amplification of a plasmid containing a poly(A) structure, but they are not always efficient. Therefore, finding more stable poly(A) fermentation strains to reduce the risk of deletion or impurity of poly(A) tail sequence without affecting plasmid output may become a new research direction. However, there are few reports on the effects of different strains on the stability of poly(A) tail sequence at present.

Gyrase is a type II DNA topoisomerase important for bacterial survival and is involved in processes such as DNA replication, repair, recombination and transcription (Menzel, R., and Gellert, M. (1994) Adv. Pharmacol. 29A:201-225). Gyrase generally composed of two GyrA subunits and two GyrB subunits. The GyrA subunit is primarily responsible for DNA binding, forming breaks on DNA double strands, and rejoining the breaks, while the GyrB subunit mainly mediates energy transduction and ATP hydrolysis.

In particular, gyrase may unwind (+) supercoils caused by replication and transcription and may introduce (-) supercoils into genomic DNA. In bacteria such as *Escherichia coli*, the gyrase is able to create a temporary break in one segment (G segment) of the DNA double strand, allowing another segment (T segment) to move to the front of the G segment via this break, thereby resulting a conversion of DNA from (+) supercoil to (-) supercoil. The removal of the (+) supercoil is a prerequisite for the progression of a replication fork and is also necessary for separation of the two double strands produced by replication (Nöllmann M, et al.,(2007) Biochimie. 89(4):490-499).

At every moment, at least 300 DNA gyrase molecules are stably bound to the genome of *Escherichia coli*, with an average of about 12 gyrases on each replication fork (Stracy M, et al.,(2019) Nucleic Acids Res.47(1):210-220). When gyrase is inhibited, the transcriptional activity of most genes decreases. For example, when the GyrI that inhibits the gyrase activity is overexpressed, the strain growth will be inhibited (Nakanishi A, et al.,(2002) J Biol Chem.277(11):8949-54). Some antibacterial drugs, such as quinolone antibiotics and coumarin drugs, are present with the gyrase as the target, causing irreversible damage to bacterial DNA (Maxwell, A. (1993) Mol. Microbiol. 9:681-686; Maxwell, A. (1997) Trends Microbiol.5:102-109).

### Summary of the Invention

The inventors of the present application have surprisingly found that when the activity of gyrase is down-regulated in a host cell, particularly in an *Escherichia coli* host cell, the replication stability and/or expression stability of poly(A) sequences in plasmids may be increased while maintaining plasmid output and the supercoil ratio of plasmids.

Therefore, in a first aspect, the present application provides a host cell comprising a down-regulated gyrase activity, or a down-regulated gyrase expression level. In particular, the present application provides a recombinant host cell, which has been engineered to comprise a down-regulated gyrase activity, or a down-regulated gyrase expression level. The gyrase comprises GyrA and GyrB. In particular, the gyrase consists of GyrA and GyrB. A host cell, including a recombinant host cell, may comprise a down-regulated GyrA activity, GyrB activity, or activity of gyrase consisting of GyrA and GyrB. A host cell, including a recombinant host cell, may comprise a down-regulated GyrA expression level, GyrB expression level, or expression level of gyrase consisting of GyrA and GyrB.

A host cell, including a recombinant host cell, may comprise a down-regulated gyrase activity due to a mutation in the *gyrA* gene or the *gyrB* gene.

In some embodiments, the host cell may comprise a down-regulated gyrase activity due to a mutation in the *gyrA* gene. A host cell comprising a mutated *gyrA* gene may express a GyrA mutant. Compared with the expression product of the *gyrA* gene without the mutation, the GyrA mutant may cause down-regulation or reduction of gyrase activity. A host cell may express a GyrA mutant due to a mutation in the *gyrA* gene. The GyrA mutant may comprise a mutation at position 80 corresponding to SEQ ID NO. 37, or mutations at positions 569 and 586 corresponding to SEQ ID NO. 37. In some embodiments, the host cell may express a GyrA mutant due to a mutation in the *gyrA* gene. The GyrA mutant may comprise an H80A mutation at position 80 corresponding to SEQ ID NO. 37, or A569T and T586A mutations at positions 569 and 586 corresponding to SEQ ID NO. 37. In some embodiments, the *Escherichia coli* host cell expresses a GyrA mutant due to a mutation in the *gyrA* gene. The GyrA mutant comprises: i) an H80A mutation at position 80 corresponding to SEQ ID NO. 37, or ii) A569T and T586A mutations at positions 569 and 586 corresponding to SEQ ID NO. 37. In some embodiments, the GyrA mutant may comprise an H80A mutation at position 80 corresponding to SEQ ID NO. 37. In other embodiments, the GyrA mutant may comprise A569T and T586A mutations at positions 569 and 586 corresponding to SEQ ID NO. 37. In some specific embodiments, the GyrA mutant may comprise: i) an H80A mutation at position 80 corresponding to SEQ ID NO. 37; or ii) A569T and T586A mutations at positions 569 and 586 corresponding to SEQ ID NO. 37. In some embodiments, the GyrA mutant may comprise an amino acid sequence as set forth in SEQ ID NO. 38, wherein the amino acids at positions 80, 569, and 586 are A, A, and T, respectively. In some embodiments, the GyrA mutant may comprise an amino acid sequence as set forth in SEQ ID NO. 38, wherein the amino acids at positions 80, 569, and 586 are A, T, and A, respectively. In some embodiments, the mutated *gyrA* gene may comprise a nucleotide sequence as set forth in SEQ ID NO. 3. In some embodiments, the mutated *gyrA* gene may comprise a nucleotide sequence as set forth in SEQ ID NO. 4.

In some embodiments, the host cell may comprise a down-regulated gyrase activity due to a mutation in the *gyrB* gene. A host cell comprising a mutated *gyrB* gene may express a GyrB mutant. Compared with the expression product of the *gyrB* gene without the mutation, the GyrB mutant may cause down-regulation of gyrase activity. A host cell may express a GyrB mutant due to a mutation in the *gyrB* gene. The GyrB mutant may comprise an E42D mutation at position 42 corresponding to SEQ ID NO. 39, an R136C mutation at position 136 corresponding to SEQ ID NO. 39, or a D498A mutation at position 498 corresponding to SEQ ID NO. 39. In some embodiments, the *Escherichia coli* host cell expresses a GyrB mutant due to a mutation in the *gyrB* gene, the GyrB mutant may comprise: i) an R136C mutation at position 136 corresponding to SEQ ID NO. 39, ii) an E42D mutation at position 42 corresponding to SEQ ID NO. 39, or iii) a D498A mutation at position 498 corresponding to SEQ ID NO. 39. In some embodiments, the GyrB mutant may comprise an R136C mutation at position 136 corresponding to SEQ ID NO. 39. In other embodiments, the GyrB mutant may comprise an E42D mutation at position 42 corresponding to SEQ ID NO. 39. In some embodiments, the GyrB mutant may comprise an D498A mutation at position 498 corresponding to SEQ ID NO. 39. In some specific embodiments, the GyrB mutant may comprise: i) an R136C mutation at position 136 corresponding to SEQ ID NO. 39, ii) an E42D mutation at position 42 corresponding to SEQ ID NO. 39, or iii) a D498A mutation at position 498 corresponding to SEQ ID NO. 39. In some embodiments, the GyrB mutant may comprise an amino acid sequence as set forth in SEQ ID NO. 40, wherein the amino acids at positions 42, 136, and 498 are D, R, and D, respectively. In some embodiments, the GyrB mutant may comprise an amino acid sequence as set forth in SEQ ID NO. 40, wherein the amino acids at positions 42, 136, and 498 are E, C, and D, respectively. In some embodiments, the GyrB mutant may comprise an amino acid sequence as set forth in SEQ ID NO. 40, wherein the amino acids at positions 42, 136, and 498 are E, R, and A, respectively. In some embodiments, the mutated *gyrB* gene may comprise a nucleotide sequence as set forth in SEQ ID NO. 5. In some embodiments, the mutated *gyrB* gene may comprise a nucleotide sequence as set forth in SEQ ID NO. 6. In some embodiments, the mutated *gyrB* gene may comprise a nucleotide sequence as set forth in SEQ ID NO. 7.

In some embodiments, the host cell may comprise a down-regulated gyrase activity due to mutations in the *gyrA* gene and the *gyrB* gene. A GyrA mutant may be expressed from the *gyrA* gene comprising a mutation, and a GyrB mutant may be expressed from the *gyrB* gene comprising a mutation. The gyrase formed by the GyrA mutant and the GyrB mutant may have a down-regulated gyrase activity compared with the gyrase formed by the expression products of the *gyrA* gene and the *gyrB* gene without these mutations in host cells. In some embodiments, the GyrA mutant may comprise an H80A mutation at position 80 corresponding to SEQ ID NO. 37, and the GyrB mutant may comprise an R136C mutation at position 136 corresponding to SEQ ID NO. 39. In some embodiments, the GyrA mutant may comprise an amino acid sequence as set forth in SEQ ID NO. 38, wherein the amino acids at positions 80, 569, and 586 are A, A, and T, respectively. The GyrB mutant may comprise an amino acid sequence as set forth in SEQ ID NO. 40, wherein the amino acids at positions 42, 136, and 498 are E, C, and D, respectively. In some embodiments, the mutated *gyrA* gene may comprise a nucleotide sequence as set forth in SEQ ID NO. 3, and the mutated *gyrB* gene may comprise a nucleotide sequence as set forth in SEQ ID NO. 6.

In some embodiments, the host cell may further include one or more of the following mutations: i) a mutation in a *RecA* gene, ii) an insertion of a *lacI* gene expression cassette, and iii) a knockout of a *RecQ* gene. In some embodiments, the host cell may include: i) a mutation in a *RecA* gene, ii) an insertion of a *lacI* gene expression cassette, and iii) a knockout of a *RecQ* gene. In other embodiments, the host cell may include: i) a mutation in a *RecA* gene, wherein the mutated *RecA* gene comprising a nucleotide sequence as set forth in SEQ ID NO: 44, ii) an insertion of a *lacI* gene expression cassette, wherein the inserted *lacI* gene expression cassette comprising a nucleotide sequence as set forth in SEQ ID NO: 42, or iii) a knockout of a *RecQ* gene, wherein the *RecQ* gene comprising a nucleotide sequence as set forth in SEQ ID NO: 41. In some embodiments, the host cell may include a mutation in a *RecA* gene, and the mutated *RecA* gene comprises a nucleotide sequence as set forth in SEQ ID NO. 44. In other embodiments, the host cell may include an insertion of a *lacI* gene expression cassette, and the inserted *lacI* gene expression cassette comprises a nucleotide sequence as set forth in SEQ ID NO. 42. In some embodiments, the host cell may include a knockout of a *RecQ* gene, and the *RecQ* gene comprises a nucleotide sequence as set forth in SEQ ID NO. 41. In some specific embodiments, the *Escherichia coli* host cell may include:
i) a mutation in a *RecA* gene, wherein the mutated *RecA* gene comprising a nucleotide sequence as set forth in SEQ ID NO. 44,
ii) an insertion of a *lacI* gene expression cassette, wherein the inserted *lacI* gene expression cassette comprising a nucleotide sequence as set forth in SEQ ID NO. 42, or/and
iii) a knockout of a *RecQ* gene, wherein the *RecQ* gene comprising a nucleotide sequence as set forth in SEQ ID NO. 41.

In other specific embodiments, the *Escherichia coli* host cell may include:
i) the mutation in the *gyrA* gene, wherein the mutated *gyrA* gene comprising a nucleotide sequence as set forth in SEQ ID NO: 3 or 4,
ii) the mutation in the *RecA* gene, wherein the mutated *RecA* gene comprising a nucleotide sequence as set forth in SEQ ID NO. 44,
iii) an insertion of a *lacI* gene expression cassette, wherein the inserted *lacI* gene expression cassette comprising a nucleotide sequence as set forth in SEQ ID NO. 42, and
iv) the knockout of the *RecQ* gene, wherein the *RecQ* gene comprising a nucleotide sequence as set forth in SEQ ID NO. 41.

A host cell, including a recombinant host cell, may comprise a down-regulated gyrase expression level through a knockdown of a *gyrA* gene or a *gyrB* gene, or a knockout of the *gyrA* gene or the *gyrB* gene combined with an expression of a GyrA/GyrB vector.

A host cell may comprise a down-regulated gyrase expression level through a knockdown of a *gyrA* gene, or a knockout of the *gyrA* gene combined with introduction of a vector expressing GyrA into the host cell. The knockdown of the *gyrA* gene may be achieved by providing an inhibitory nucleic acid molecule (e.g., shRNA) targeting GyrA. The recombinant host cell may comprise a down-regulated gyrase expression level through a knockdown of a *gyrB* gene, or a knockout of the *gyrB* gene combined with introduction of a vector expressing GyrB into the host cell. The knockdown of the *gyrB* gene may be achieved by providing an inhibitory nucleic acid molecule (e.g., shRNA) targeting GyrB.

The host cell may be an *Escherichia coli* host cell. In particular, the host cell, particularly the recombinant host cell, may be JM108, NEB Stable, Top10, DH5α, DH10B, MG1655, AG1, BL21, DB3.1, DC10B, DH1, E.Cloni 10G, EPI300, EPI400, JM109, JM110, STBL2, STBL3, SURE, TOP10F', XL1-Blue or XL10-Gold that has been engineered to comprise a down-regulated gyrase activity or a down-regulated gyrase expression level. In some embodiments, the recombinant host cell may be JM108, NEB Stable, Top10, DH5α, DH10B, or MG1655.

A host cell, including a recombinant host cell, may comprise a nucleic acid molecule containing a poly(A) sequence. For example, the host cell may comprise a vector, and the vector may comprise the nucleic acid molecule containing the poly(A) sequence. Alternatively, the host cell may have the nucleic acid molecule containing the poly(A) sequence integrated into its genome. The poly(A) sequence may comprise consecutive A bases, and the number of consecutive A bases may be, for example, 20-250. The poly(A) sequence may comprise 2-5 consecutive A base stretches separated by a non-A base, wherein the number of consecutive A bases in each consecutive A base stretch may be, for example, 10-100, and the consecutive A base stretches may be separated by, for example, 1-20 non-A bases.

A host cell, including a recombinant host cell, may also comprise a nucleic acid molecule containing a poly(T) sequence.

In a second aspect, the present application provides a method for preparing a recombinant host cell, comprising:
i) providing a host cell comprising a *gyrA* gene and a *gyrB* gene, such as a prokaryotic host cell, and
ii) down-regulating the activity of a gyrase in the host cell or down-regulating the expression level of the gyrase in the host cell.

The host cell in step i) may be an *Escherichia coli* host cell, particularly an *Escherichia coli* strain for vector preparation, including, but not limited to, JM108, NEB Stable, Top10, DH5α, DH10B, MG1655, AG1, BL21, DB3.1, DC10B, DH1, E.Cloni 10G, EPI300, EPI400, JM109, JM110, STBL2, STBL3, SURE, TOP10F', XL1-Blue and XL10-Gold.

The gyrase comprises GyrA and GyrB. In particular, the gyrase consists of GyrA and GyrB. Step ii) may include down-regulating the activity of GyrA, the activity of GyrB, or the activity of gyrase consisting of GyrA and GyrB. Step ii) may include down-regulating the expression level of GyrA, the expression level of GyrB, or the expression level of gyrase consisting of GyrA and GyrB.

Down-regulating the gyrase activity in step ii) may be achieved through a mutation in a *gyrA* gene or a *gyrB* gene.

In some embodiments, down-regulating the gyrase activity may be achieved through a mutation in a *gyrA* gene. An *Escherichia coli* host cell comprising a mutated *gyrA* gene may express a GyrA mutant. Compared with the expression product of the *gyrA* gene without the mutation, the GyrA mutant may cause down-regulation of gyrase activity. The GyrA mutant may comprise an H80A mutation at position 80 corresponding to SEQ ID NO. 37, or A569T and T586A mutations at positions 569 and 586 corresponding to SEQ ID NO. 37. In some embodiments, the GyrA mutant may comprise an amino acid sequence as set forth in SEQ ID NO. 38, wherein the amino acids at positions 80, 569, and 586 are A, A, and T, respectively. In some embodiments, the GyrA mutant may comprise an amino acid sequence as set forth in SEQ ID NO. 38, wherein the amino acids at positions 80, 569, and 586 are A, T, and A, respectively. In some embodiments, the mutated *gyrA* gene may comprise a nucleotide sequence as set forth in SEQ ID NO. 3. In some embodiments, the mutated *gyrA* gene may comprise a nucleotide sequence as set forth in SEQ ID NO. 4.

In some embodiments, down-regulating the gyrase activity may be achieved through a mutation in a *gyrB* gene. An *Escherichia coli* host cell comprising a mutated *gyrB* gene may express a GyrB mutant. Compared with the expression product of the *gyrB* gene without the mutation, the GyrB mutant may cause down-regulation of gyrase activity. The GyrB mutant may comprise an E42D mutation at position 42 corresponding to SEQ ID NO. 39, an R136C mutation at position 136 corresponding to SEQ ID NO. 39, or a D498A mutation at position 498 corresponding to SEQ ID NO. 39. In some embodiments, the GyrB mutant may comprise an amino acid sequence as set forth in SEQ ID NO. 40, wherein the amino acids at positions 42, 136, and 498 are D, R, and D, respectively. In some embodiments, the GyrB mutant may comprise an amino acid sequence as set forth in SEQ ID NO. 40, wherein the amino acids at positions 42, 136, and 498 are E, C, and D, respectively. In some embodiments, the GyrB mutant may comprise an amino acid sequence as set forth in SEQ ID NO. 40, wherein the amino acids at positions 42, 136, and 498 are E, R, and A, respectively. In some embodiments, the mutated *gyrB* gene may comprise a nucleotide sequence as set forth in SEQ ID NO. 5. In some embodiments, the mutated *gyrB* gene may comprise a nucleotide sequence as set forth in SEQ ID NO. 6. In some embodiments, the mutated *gyrB* gene may comprise a nucleotide sequence as set forth in SEQ ID NO. 7.

In some embodiments, down-regulating the gyrase activity may be achieved through mutations in a *gyrA* gene and a *gyrB* gene. A GyrA mutant may be expressed from the *gyrA* gene comprising a mutation, and a GyrB mutant may be expressed from the *gyrB* gene comprising a mutation. The gyrase formed by the GyrA mutant and the GyrB mutant may have a down-regulated gyrase activity compared with the gyrase formed by the expression products of the gyrA gene and the *gyrB* gene without these mutations in *Escherichia coli* host cells. In some embodiments, the GyrA mutant may comprise an H80A mutation at position 80 corresponding to SEQ ID NO. 37, and the GyrB mutant may comprise an R136C mutation at position 136 corresponding to SEQ ID NO. 39. In some embodiments, the GyrA mutant may comprise an amino acid sequence as set forth in SEQ ID NO. 38, wherein the amino acids at positions 80, 569, and 586 are A, A, and T, respectively. The GyrB mutant may comprise an amino acid sequence as set forth in SEQ ID NO. 40, wherein the amino acids at positions 42, 136, and 498 are E, C, and D, respectively. In some embodiments, the mutated *gyrA* gene may comprise a nucleotide sequence as set forth in SEQ ID NO. 3, and the mutated *gyrB* gene may comprise a nucleotide sequence as set forth in SEQ ID NO. 6.

In some embodiments, step ii) may include,
1) engineering the *gyrA* gene to comprise a nucleotide sequence as set forth in SEQ ID NO. 3,
2) engineering the *gyrB* gene to comprise a nucleotide sequence as set forth in SEQ ID NO. 6,
3) engineering the *gyrA* gene to comprise a nucleotide sequence as set forth in SEQ ID NO. 4,
4) engineering the *gyrB* gene to comprise a nucleotide sequence as set forth in SEQ ID NO. 5, or
5) engineering the *gyrB* gene to comprise a nucleotide sequence as set forth in SEQ ID NO. 7.

In some embodiments, step ii) may include introducing into the host cell of step i) a Cas9 enzyme, and
1) an sgRNA comprising a nucleotide sequence as set forth in SEQ ID NO. 8 and a donor sequence comprising a nucleotide sequence as set forth in SEQ ID NO. 13,
2) an sgRNA comprising a nucleotide sequence as set forth in SEQ ID NO. 9 and a donor sequence comprising a nucleotide sequence as set forth in SEQ ID NO. 14,
3) an sgRNA comprising a nucleotide sequence as set forth in SEQ ID NO. 10 and a donor sequence comprising a nucleotide sequence as set forth in SEQ ID NO. 15,
4) an sgRNA comprising a nucleotide sequence as set forth in SEQ ID NO. 11 and a donor sequence comprising a nucleotide sequence as set forth in SEQ ID NO. 16, or
5) an sgRNA comprising a nucleotide sequence as set forth in SEQ ID NO. 12 and a donor sequence comprising a nucleotide sequence as set forth in SEQ ID NO. 17.

The gene encoding the Cas9 enzyme may be in a vector. The sgRNA and the donor sequence may be in a vector. Step ii) may include, introducing a vector containing a gene encoding Cas9 enzyme, and a vector containing sgRNA and a donor sequence into the host cell of step i).

Down-regulating the gyrase expression level in step ii) may be achieved through a knockdown of a *gyrA* gene or a *gyrB* gene, or a knockout of the *gyrA* gene or the *gyrB* gene combined with introduction of a vector expressing GyrA/GyrB into the host cell.

Down-regulating the gyrase expression level in step ii) may be achieved through a knockdown of a *gyrA* gene, or a knockout of the *gyrA* gene combined with introduction of a vector expressing GyrA into the host cell. The knockdown of the *gyrA* gene may be achieved by providing an inhibitory nucleic acid molecule (e.g., shRNA) targeting GyrA. Down-regulating the gyrase expression level in step ii) may be achieved through a knockdown of a *gyrB* gene, or a knockout of the *gyrB* gene combined with introduction of a vector expressing GyrB into the host cell. The knockdown of the *gyrB* gene may be achieved by providing an inhibitory nucleic acid molecule (e.g., shRNA) targeting GyrB.

In some embodiments, the *Escherichia coli* host cell may further include any one or more of the following operation steps: 1) mutating a *RecA* gene, 2) inserting a *lacI* gene expression cassette, and 3) knocking out a *RecQ* gene. In some embodiments, the *Escherichia coli* host cell may include: 1) a mutation in a *RecA* gene, 2) an insertion of a *lacI* gene expression cassette, and 3) a knockout of a *RecQ* gene.

In some embodiments, step ii) may include introducing into the host cell a Cas9 enzyme, and
1) an sgRNA comprising a nucleotide sequence as set forth in SEQ ID NO. 47 and a donor sequence comprising a nucleotide sequence as set forth in SEQ ID NO. 50,
2) an sgRNA comprising a nucleotide sequence as set forth in SEQ ID NO. 46 and a donor sequence comprising a nucleotide sequence as set forth in SEQ ID NO. 49, or
3) an sgRNA comprising a nucleotide sequence as set forth in SEQ ID NO. 45 and a donor sequence comprising a nucleotide sequence as set forth in SEQ ID NO. 48.

In a third aspect, the present application provides the use of a host cell with a down-regulated gyrase activity or a down-regulated gyrase expression level, particularly a prokaryotic host cell, comprising the host cell (including the recombinant host cell) of the present application, and the recombinant host cell obtained by the preparation method of the present application, in the replication or expression of a nucleic acid molecule containing a poly(A) sequence or a poly(T) sequence.

The host cell according to the use of the present application may be an *Escherichia coli* host cell. In particular, the host cell according to the use of the present application may be JM108, NEB Stable, Top10, DH5α, DH10B, MG1655, AG1, BL21, DB3.1, DC10B, DH1, E.Cloni 10G, EPI300, EPI400, JM109, JM110, STBL2, STBL3, SURE, TOP10F', XL1-Blue or XL10-Gold that has been engineered to comprise a down-regulated gyrase activity or a down-regulated gyrase expression level. In some embodiments, the host cell according to the use of the present application may be JM108, NEB Stable, Top10, DH5α, DH10B, or MG1655 that has been engineered to comprise a down-regulated gyrase activity or a down-regulated gyrase expression level.

The host cell according to the use of the present application, in some embodiments, comprises a mutation in a *gyrA* gene or a *gyrB* gene that results in a decreased gyrase activity. A host cell comprising a mutated *gyrA* gene may express a GyrA mutant. The GyrA mutant may comprise an H80A mutation at position 80 corresponding to SEQ ID NO. 37, or A569T and T586A mutations at positions 569 and 586 corresponding to SEQ ID NO. 37. In some embodiments, the GyrA mutant may comprise an amino acid sequence as set forth in SEQ ID NO. 38, wherein the amino acids at positions 80, 569, and 586 are A, A, and T, respectively. In some embodiments, the GyrA mutant may comprise an amino acid sequence as set forth in SEQ ID NO. 38, wherein the amino acids at positions 80, 569, and 586 are A, T, and A, respectively. In some embodiments, the mutated *gyrA* gene may comprise a nucleotide sequence as set forth in SEQ ID NO. 3. In some embodiments, the mutated *gyrA* gene may comprise a nucleotide sequence as set forth in SEQ ID NO. 4. A host cell comprising a mutated *gyrB* gene may express a GyrB mutant. The GyrB mutant may comprise an E42D mutation at position 42 corresponding to SEQ ID NO. 39, an R136C mutation at position 136 corresponding to SEQ ID NO. 39, or a D498A mutation at position 498 corresponding to SEQ ID NO. 39. In some embodiments, the GyrB mutant may comprise an amino acid sequence as set forth in SEQ ID NO. 40, wherein the amino acids at positions 42, 136, and 498 are D, R, and D, respectively. In some embodiments, the GyrB mutant may comprise an amino acid sequence as set forth in SEQ ID NO. 40, wherein the amino acids at positions 42, 136, and 498 are E, C, and D, respectively. In some embodiments, the GyrB mutant may comprise an amino acid sequence as set forth in SEQ ID NO. 40, wherein the amino acids at positions 42, 136, and 498 are E, R, and A, respectively. In some embodiments, the mutated *gyrB* gene may comprise a nucleotide sequence as set forth in SEQ ID NO. 5. In some embodiments, the mutated *gyrB* gene may comprise a nucleotide sequence as set forth in SEQ ID NO. 6. In some embodiments, the mutated *gyrB* gene may comprise a nucleotide sequence as set forth in SEQ ID NO. 7. In some embodiments, the host cell may comprise mutations in the *gyrA* gene and the *gyrB* gene that result in a decreased gyrase activity. A GyrA mutant may be expressed from the *gyrA* gene comprising a mutation, and a GyrB mutant may be expressed from the *gyrB* gene comprising a mutation. The GyrA mutant may comprise an H80A mutation at position 80 corresponding to SEQ ID NO. 37, and the GyrB mutant may comprise an R136C mutation at position 136 corresponding to SEQ ID NO. 39. The host cell according to the use of the present application, in some embodiments, may comprise a down-regulated gyrase expression level through a knockdown of a *gyrA* gene or a *gyrB* gene, or a knockout of the *gyrA* gene or the *gyrB* gene combined with an expression of a GyrA/GyrB vector. For example, the host cell may comprise a down-regulated gyrase expression level through a knockdown of a *gyrA* gene, or a knockout of the *gyrA* gene combined with introduction of a vector expressing GyrA into the host cell. The knockdown of the gyrA gene may be achieved by providing an inhibitory nucleic acid molecule (e.g., shRNA) targeting GyrA. The host cell may comprise a down-regulated gyrase expression level through a knockdown of a *gyrB* gene, or a knockout of the *gyrB* gene combined with introduction of a vector expressing GyrB into the host cell. The knockdown of the *gyrB* gene may be achieved by providing an inhibitory nucleic acid molecule (e.g., shRNA) targeting GyrB.

The host cell according to the use of the present application may further include one or more of the following mutations: i) a mutation in a *RecA* gene, ii) an insertion of a *lacI* gene expression cassette, and iii) a knockout of a *RecQ* gene. In some embodiments, the host cell may include: i) a mutation in a *RecA* gene, ii) an insertion of a *lacI* gene expression cassette, and iii) a knockout of a *RecQ* gene. In other embodiments, the host cell may include: i) a mutation in a *RecA* gene, wherein the mutated *RecA* gene comprising a nucleotide sequence as set forth in SEQ ID NO: 44, ii) an insertion of a *lacI* gene expression cassette, wherein the inserted *lacI* gene expression cassette comprising a nucleotide sequence as set forth in SEQ ID NO: 42, or iii) a knockout of a *RecQ* gene, wherein the *RecQ* gene comprising a nucleotide sequence as set forth in SEQ ID NO: 41. In some embodiments, the host cell may include a mutation in a *RecA* gene, wherein the mutated *RecA* gene expresses a protein with a decreased activity compared to the protein expressed by the wild-type *RecA* (e.g., a recombination-deficient mutation), and the mutated *RecA* gene may be *RecA1*. In some specific embodiments, the host cell may include a mutation in a *RecA* gene, and the mutated *RecA* gene comprises a nucleotide sequence as set forth in SEQ ID NO. 44. In other embodiments, the host cell may include an insertion of a *lacI* gene expression cassette, which includes a *lacIq* promoter, *lacI* and/or a terminator. In some specific embodiments, the inserted *lacI* gene expression cassette comprises a nucleotide sequence as set forth in SEQ ID NO. 42. In some embodiments, the host cell may include a knockout of a *RecQ* gene, and the *RecQ* gene comprises a nucleotide sequence as set forth in SEQ ID NO. 41.

In some specific embodiments, the *Escherichia coli* host cell may include:
i) a mutation in a *RecA* gene, wherein the mutated *RecA* gene comprising a nucleotide sequence as set forth in SEQ ID NO. 44,
ii) an insertion of a *lacI* gene expression cassette, wherein the inserted *lacI* gene expression cassette comprising a nucleotide sequence as set forth in SEQ ID NO. 42, or/and
iii) a knockout of a *RecQ* gene, wherein the *RecQ* gene comprising a nucleotide sequence as set forth in SEQ ID NO. 41. A nucleic acid molecule containing a poly(A) sequence may be contained in a vector, and the vector is contained in a host cell, or may also be integrated into the genome of the host cell. The poly(A) sequence may comprise consecutive A bases, and the number of consecutive A bases may be, for example, 20-250. The poly(A) sequence may comprise 2-5 consecutive A base stretches separated by a non-A base, wherein the number of consecutive A bases in each consecutive A base stretch may be, for example, 10-100, and the consecutive A base stretches may be separated by, for example, 1-20 non-A bases.

In a fourth aspect, the present application provides a method for replicating or expressing a nucleic acid molecule containing a poly(A) sequence or a poly(T) sequence using a host cell comprising a down-regulated gyrase activity or a down-regulated gyrase expression level, particularly a prokaryotic host cell, including the host cell of the present application, and the recombinant host cell prepared and obtained by the method of the present application.

The method for replicating the nucleic acid molecule containing the poly(A) sequence may include:
i) introducing a vector into the host cell, wherein the vector comprises the nucleic acid molecule containing the poly(A) sequence, and
ii) culturing the host cell under a condition that facilitates replication of the vector.

The method for expressing the nucleic acid molecule containing the poly(A) sequence may include:
i) introducing a vector into the host cell, wherein the vector comprises the nucleic acid molecule containing the poly(A) sequence,
ii) optionally, culturing the host cell under a condition that facilitates replication of the vector, and
iii) culturing the host cell under a condition that facilitates expression of the vector, or
extracting the vector from the host cell of step i) or ii) and performing in vitro transcription on the vector.

The method for expressing the nucleic acid molecule containing the poly(A) sequence may include:
i) integrating the nucleic acid molecule containing the poly(A) sequence into the genome of the host cell, and
ii) culturing the host cell under a condition that facilitates expression of the nucleic acid molecule containing the poly(A) sequence.

The host cell may be an *Escherichia coli* host cell. In particular, the host cell may be JM108, NEB Stable, Top10, DH5α, DH10B, MG1655, AG1, BL21, DB3.1, DC10B, DH1, E.Cloni 10G, EPI300, EPI400, JM109, JM110, STBL2, STBL3, SURE, TOP10F', XL1-Blue or XL10-Gold that has been engineered to comprise a down-regulated gyrase activity or a down-regulated gyrase expression level. In some embodiments, the host cell may be JM108, NEB Stable, Top10, DH5α, DH10B, or MG1655 that has been engineered to comprise a down-regulated gyrase activity or a down-regulated gyrase expression level.

A host cell, including a recombinant host cell, in some embodiments, may comprise a mutation in a *gyrA* gene or a *gyrB* gene that results in a decreased gyrase activity. A host cell comprising a mutated *gyrA* gene may express a GyrA mutant. The GyrA mutant may comprise an H80A mutation at position 80 corresponding to SEQ ID NO. 37, or A569T and T586A mutations at positions 569 and 586 corresponding to SEQ ID NO. 37. In some embodiments, the GyrA mutant may comprise an amino acid sequence as set forth in SEQ ID NO. 38, wherein the amino acids at positions 80, 569, and 586 are A, A, and T, respectively. In some embodiments, the GyrA mutant may comprise an amino acid sequence as set forth in SEQ ID NO. 38, wherein the amino acids at positions 80, 569, and 586 are A, T, and A, respectively. In some embodiments, the mutated *gyrA* gene may comprise a nucleotide sequence as set forth in SEQ ID NO. 3. In some embodiments, the mutated *gyrA* gene may comprise a nucleotide sequence as set forth in SEQ ID NO. 4. A host cell comprising a mutated *gyrB* gene may express a GyrB mutant. The GyrB mutant may comprise an E42D mutation at position 42 corresponding to SEQ ID NO. 39, an R136C mutation at position 136 corresponding to SEQ ID NO. 39, or a D498A mutation at position 498 corresponding to SEQ ID NO. 39. In some embodiments, the GyrB mutant may comprise an amino acid sequence as set forth in SEQ ID NO. 40, wherein the amino acids at positions 42, 136, and 498 are D, R, and D, respectively. In some embodiments, the GyrB mutant may comprise an amino acid sequence as set forth in SEQ ID NO. 40, wherein the amino acids at positions 42, 136, and 498 are E, C, and D, respectively. In some embodiments, the GyrB mutant may comprise an amino acid sequence as set forth in SEQ ID NO. 40, wherein the amino acids at positions 42, 136, and 498 are E, R, and A, respectively. In some embodiments, the mutated *gyrB* gene may comprise a nucleotide sequence as set forth in SEQ ID NO. 5. In some embodiments, the mutated *gyrB* gene may comprise a nucleotide sequence as set forth in SEQ ID NO. 6. In some embodiments, the mutated *gyrB* gene may comprise a nucleotide sequence as set forth in SEQ ID NO. 7. In some embodiments, the host cell may comprise mutations in the *gyrA* gene and the *gyrB* gene that result in a decreased gyrase activity. A GyrA mutant may be expressed from the *gyrA* gene comprising a mutation, and a GyrB mutant may be expressed from the *gyrB* gene comprising a mutation. The GyrA mutant may comprise an H80A mutation at position 80 corresponding to SEQ ID NO. 37, and the GyrB mutant may comprise an R136C mutation at position 136 corresponding to SEQ ID NO. 39.

A host cell, including a recombinant host cell, in some embodiments, may comprise a down-regulated gyrase expression level through a knockdown of a *gyrA* gene or a *gyrB* gene, or a knockout of the *gyrA* gene or the *gyrB* gene combined with an expression of a GyrA/GyrB vector. For example, the host cell may comprise a down-regulated gyrase expression level through a knockdown of a *gyrA* gene, or a knockout of the *gyrA* gene combined with introduction of a vector expressing GyrA into the host cell. The knockdown of the gyrA gene may be achieved by providing an inhibitory nucleic acid molecule (e.g., shRNA) targeting GyrA. The host cell may comprise a down-regulated gyrase expression level through a knockdown of a *gyrB* gene, or a knockout of the *gyrB* gene combined with introduction of a vector expressing GyrB into the host cell. The knockdown of the *gyrB* gene may be achieved by providing an inhibitory nucleic acid molecule (e.g., shRNA) targeting GyrB.

The poly(A) sequence may comprise consecutive A bases, and the number of consecutive A bases may be, for example, 20-250. The poly(A) sequence may comprise 2-5 consecutive A base stretches separated by a non-A base, wherein the number of consecutive A bases in each consecutive A base stretch may be, for example, 10-100, and the consecutive A base stretches may be separated by, for example, 1-20 non-A bases.

Other features and advantages of the present disclosure will become more apparent on the basis of the following detailed description and examples, which should not be construed as limiting. The content of all documents, Genbank records, patents, and published patent applications cited in the present application is expressly incorporated herein by reference.

### Brief Description of the Drawings

The following specific descriptions are provided by way of examples but are not intended to limit the present invention to the stated specific embodiments, and a better understanding thereof can be achieved in conjunction with the accompanying drawings.
FIGs. 1A and 1B show maps of the target (all-in-one) plasmid (FIG. 1A) and the Cas9 protein expression plasmid (FIG. 1B) used for gene mutations in *Escherichia coli*.
FIG. 2 shows a map of a test plasmid containing a poly(A) sequence.
FIGs. 3A-3C show the Sanger sequencing chromatograms of a poly(A) sequence with qualified purity (FIG. 3A), a poly(A) sequence with unqualified purity (FIG. 3B), and poly(A) sequence deletion (FIG. 3C).
FIG. 4 shows the average supercoil ratio results for different recombinant genotype strains.

### Detailed Description of Embodiments

The terms used herein have the ordinary meaning as used in dictionaries, textbooks, and technical reference books, or as commonly understood by one of ordinary skill in the art, unless otherwise specified. The following description of certain terms is for the purpose of facilitating understanding of the present application only and is not intended to limit the terms specifically unless otherwise specified.

As used herein and in the appended claims, the singular forms "a", "an", and "the" include plural forms of the referent unless the context clearly dictates otherwise.

The term "or" refers to a single element of enumerative selectable elements unless the context clearly dictates otherwise.

The term "contain" or "comprise" means that the described elements, integers, or steps are included, but the addition of any other elements, integers, or steps is not precluded. When the term "contain" or "comprise" is used herein, unless otherwise indicated, combinations of the described elements, integers, or steps are also included.

A "poly(A) structure" or a "poly(A) sequence" refers to a nucleotide sequence comprising consecutive repeating A bases, and also includes a nucleotide sequence in which several consecutive A base stretches are separated by a non-A base. Similarly, a "poly(T) structure" or a "poly(T) sequence" refers to a nucleotide sequence comprising consecutive repeating T bases, and also includes a nucleotide sequence in which several consecutive T base stretches are separated by a non-T base.

Both "host cell" and "strain" herein refer to any cell into which an exogenous nucleic acid molecule (such as a vector) may be introduced to enable the exogenous nucleic acid molecule to be replicated or expressed. The host cell of the present application may be a prokaryotic cell, such as *Escherichia coli*. "*Escherichia coli*" and "*E.coli*" may be used interchangeably, including wild-type *Escherichia coli*, and *Escherichia coli* strains comprising artificial or natural mutations, such as JM108, NEB Stable, Top10, DH5α or DH10B strains, and the like.

"Supercoil" includes (+) supercoil and (-) supercoil, which are the helical states of the DNA strands. The main difference between the (+) supercoil and (-) supercoil of DNA is that during the (+) supercoiling of DNA, the DNA strand is over-wound compared to the relaxed state, while during the (-) supercoiling of DNA, the DNA strand is in a wound state compared to the relaxed state. In most organisms, DNA exists in the (-) supercoiled state under normal conditions, and the (+) supercoil occurs only during specific cellular functions.

"Gyrase", also known as DNA gyrase, or rotase, is a type of helicase, specifically a topoisomerase II. This enzyme may introduce a (-) supercoil into DNA and convert a (+) supercoil to a (-) supercoil, and participate in important processes such as replication, transcription, repair and recombination in prokaryotic cells. Gyrase consists of the GyrA subunit and the GyrB subunit. The GyrA is primarily responsible for forming breaks on DNA double strands, and rejoining the breaks, while the GyrB subunit mainly mediates energy transduction and ATP hydrolysis. GyrA is encoded by a *gyrA* gene, and GyrB is encoded by a *gyrB* gene. It is known that mutations such as the H80A mutation, and the A569T and T586A double mutation in GyrA, and the E42D mutation, the R136C mutation, and the D498A mutation in GyrB may result in a decreased gyrase activity (Hockings SC, Maxwell A. (2002) J Mol Biol. 318(2):351-9; Oram M, Fisher LM.(1992) J Bacteriol. 174(12):4175-8; Gross CH et al., (2003) Antimicrob Agents Chemother. 47(3):1037-46; Contreras A, Maxwell A. (1992) Mol Microbiol. 6(12):1617-24; Noble CG, Maxwell A. (2002) J Mol Biol.318(2):361-71).

As used herein, "stability" refers to structural (base) integrity of sequences such as poly(A) and poly(T), purity of structural genes, and the like. "Integrity" refers to the consistency between the poly(A) sequence that is replicated or expressed in a strain and the poly(A) sequence that is originally constructed and transformed into the strain.

Generally speaking, the "down-regulate" refers to a reduction. For example, "down-regulating" gyrase activity refers to reducing the activity of GyrA, GyrB, or the gyrase consisting of both, including the activity of converting (+) supercoils to (-) supercoils, by a mutation of a *gyrA* gene and/or a *gyrB* gene, increased expression of GyrI, and other means. "Down-regulating" the expression level of gyrase refers to reducing the expression levels of GyrA, GyrB, or both by means such as a knockdown of a *gyrA* gene and/or a *gyrB* gene.

"Knockdown" herein refers to specific degradation of target mRNA, or interference with normal RNA translation or cleavage by certain means, such as RNAi technology and the like, thereby reducing the expression of a target gene or sequence (e.g., the *gyrA* gene and/or *gyrB* gene herein), but not completely eliminating the expression of the *gyrA* gene and/or *gyrB* gene. While "knockout" refers to the removal of a specific gene or sequence by certain means, such as homologous recombination, resulting in the complete elimination of expression of the gene or sequence.

"Introduction" refers to the introduction of a vector into a host cell by means, such as transfection, transduction, or transformation. "Integration" refers to the embedding of a certain sequence into a genome by certain means, such as homologous recombination, so that the sequence may be expressed along with the expression of the genomic sequence.

A "recombinant" cell refers to a cell obtained by altering a gene sequence, a gene expression pattern, a gene expression level, and the like by a DNA recombination technology and the like. A "recombinant" host cell refers to a cell obtained by altering a gene sequence, a gene expression pattern, a gene expression level, and the like by DNA recombination technology and the like, and into which an exogenous nucleic acid molecule may be introduced to enable the exogenous nucleic acid molecule to be replicated or expressed.

A "vector" refers to a naturally occurring or synthetic DNA fragment, including single-stranded and double-stranded DNA fragments, such as chemically synthesized DNA fragments, natural plasmids, or engineered viral genomes, and the like. An exogenous DNA fragment may be inserted into the vector for cloning and/or expression of the exogenous DNA fragment. The vector may comprise, for example, an origin of replication, a selectable marker or reporter gene, a multiple cloning site (MCS), and the like. The term includes linear DNA fragments (e.g., PCR products, linearized plasmid fragments, etc.), plasmid vectors, viral vectors, bacterial artificial chromosomes (BAC), yeast artificial chromosomes (YAC), and the like. When the vector is double-stranded DNA, the description of the order of elements and the orientation of element sequence are described relative to a single designated DNA strand.

"In vitro transcription" or "IVT" refers to a process of forming RNA in an in vitro cell-free system using DNA as a template to mimic the transcription in vivo under the condition of containing a RNA transcriptase, NTP, etc. When a plasmid vector is used as a DNA template, the plasmid is linearized by cleavage at an enzyme cleavage site prior to in vitro transcription.

During the maturation of mRNA, a poly(A) tail will be added to the end. The poly(A) tail has a great influence on the structural stability and translation efficiency of mRNA. In the current production of mRNA molecules, the coding sequence of the poly(A) tail (that is, the sequence comprising consecutive repeating A bases, or several consecutive repeating A base stretches separated by a non-A base) is usually contained in the vector. Such poly(A) tail coding sequence often undergoes significant deletion of nucleotides containing A bases along with the propagation, passage and other related processes of host cells. At present, there is no good way to prevent such deletion from happening. Moreover, since there is no natural poly(A) tail coding sequence in biological cells, it is difficult to find a good way to study the reasons for the instability of this sequence structure in vivo.

The inventors of the present application have surprisingly found that when the activity of gyrase in prokaryotic host cells (e.g., *Escherichia coli* host cell) is down-regulated, vectors containing poly(A) sequences exhibit improved replication stability in such prokaryotic host cells; that is, the loss of A bases in the poly(A) sequences during vector replication is improved.

It is well known in the art that gyrase is an important DNA helicase in prokaryotic cells, which is involved in the processes of DNA replication, repair, recombination and transcription. Drugs that target gyrase and inhibit gyrase activity, such as quinolones and coumarins, are used as antibacterial drugs, resulting in irreversible damage to bacterial DNA.

In view of the important roles of GyrA and GyrB, it is not conceivable to down-regulate the activity of GyrA, GyrB, or a gyrase composed of both in a strain for production purposes except for gene or protein research purposes, since such down-regulation is likely to cause death of the strain.

Surprisingly, however, when the activity of gyrase is down-regulated in the host cell, the host cell is able to maintain the original plasmid output. That is, by down-regulating the activity of the gyrase in the host cell, the poly(A) sequence-containing vector with high stability may be prepared at a high plasmid output. This is of a great significance for the production of a vector containing a poly(A) sequence. Simultaneously, the quality of the plasmid, such as the supercoil ratio, is also maintained at the original level.

Gyrase consists of a GyrA subunit and a GyrB subunit, with the GyrA encoded by the *gyrA* gene and the GyrB encoded by the *gyrB* gene. Studies have pointed out that H80A mutation, and the A569T and T586A double mutation in GyrA, the E42D mutation, the R136C mutation, and the D498A mutation in GyrB will cause a reduction in gyrase activity.

Using this existing information, the inventors of the present application performed corresponding mutations in the *gyrA* gene or the *gyrB* gene through gene editing in the host cell, which causes a reduction in gyrase activity.

Specifically, using a vector as the medium, Cas9 enzyme is introduced into the host cell-for example, the *Escherichia coli* host cell, particularly strains for vector construction such as JM108, NEB Stable, Top10, DH5α, and DH10B, along with i) an sgRNA comprising a nucleotide sequence as set forth in SEQ ID NO. 8 and a donor sequence comprising a nucleotide sequence as set forth in SEQ ID NO. 13, ii) an sgRNA comprising a nucleotide sequence as set forth in SEQ ID NO. 9 and a donor sequence comprising a nucleotide sequence as set forth in SEQ ID NO. 14, iii) an sgRNA comprising a nucleotide sequence as set forth in SEQ ID NO. 10 and a donor sequence as set forth in SEQ ID NO. 15, iv) an sgRNA comprising a nucleotide sequence as set forth in SEQ ID NO. 11, and a donor sequence as set forth in SEQ ID NO.16, or v) an sgRNA comprising a nucleotide sequence as set forth in SEQ ID NO. 12 and a donor sequence as set forth in SEQ ID NO.17. In the obtained host cells, the *gyrA* gene comprises a nucleotide sequence as set forth in SEQ ID NO. 3 or 4, or the *gyrB* gene comprises a nucleotide sequence as set forth in SEQ ID NO. 5, 6, or 7. A combination of the sgRNA/donor sequence described above, such as a combination of i) and iv), may also be introduced into the host cell.

When any of the host cells thus obtained is used to replicate or express a vector containing a poly(A) sequence, the replication stability of the poly(A) sequence is improved relative to the host cell without down-regulating gyrase activity. In particular, this improvement is more pronounced as the number of replications increases and the number of host cell passages increases.

Based on data from only *gyrA* mutation, only *gyrB* mutation, and *gyrA* gene + *gyrB* gene mutations, gyrase activity is influenced by both GyrA and GyrB. That is, a mutation in either GyrA or GyrB will affect the gyrase activity.

By down-regulating the expression level of gyrase in host cells, the replication and expression stability of poly(A) in the vector may also be improved.

Specifically, the expression level of gyrase in host cells may be down-regulated by means such as a knockdown of a *gyrA* gene or a *gyrB* gene, or a knockout of the *gyrA* gene or *gyrB* gene combined with an expression of a GyrA/GyrB vector. A host cell may comprise a down-regulated gyrase expression level through a knockdown of a gyrA gene, or a knockout of the gyrA gene combined with introduction of a vector expressing GyrA into the host cell. The knockdown of the gyrA gene may be achieved by providing an inhibitory nucleic acid molecule (e.g., shRNA) targeting GyrA. The recombinant host cell may comprise a down-regulated gyrase expression level through a knockdown of a *gyrB* gene, or a knockout of the *gyrB* gene combined with introduction of a vector expressing GyrB into the host cell. The knockdown of the *gyrB* gene may be achieved by providing an inhibitory nucleic acid molecule (e.g., shRNA) targeting GyrB. Knockout may be performed, for example, by means of homologous recombination, the operation of which is well known to those skilled in the art. The technology for constructing expression vectors for GyrA or GyrB and introducing them into host cells is well known to those skilled in the art.

The recombinant host cells obtained by the above methods, and recombinant host cells engineered by any other methods to comprise a down-regulated gyrase activity or a down-regulated gyrase expression level, particularly strains for recombinant vector preparation engineered to comprise a down-regulated gyrase activity or a down-regulated gyrase expression level, such as JM108, NEB Stable, Top10, DH5α, DH10B, MG1655, AG1, BL21, DB3.1, DC10B, DH1, E.Cloni 10G, EPI300, EPI400, JM109, JM110, STBL2, STBL3, SURE, TOP10F', XL1-Blue or XL10-Gold, may be used to replicate or express a nucleic acid molecule containing a poly(A) sequence. In addition, natural mutant strains comprising a down-regulated gyrase activity or a down-regulated gyrase expression level resulting from various causes, such as natural mutations in the *gyrA* gene or the *gyrB* gene, or natural knockdown of the *gyrA* gene or the *gyrB* gene, may also be used to replicate or express a nucleic acid molecule containing a poly(A) sequence.

Further, by combining mutations in other genotypes, such as *RecA*, *lacI*, and *RecQ,* the stability of replication or expression of a nucleic acid molecule containing a poly(A) sequence may be further improved. Specifically, on the basis of *Escherichia coli* host cells with only *gyrA* mutation, only *gyrB* mutation, or *gyrA* + *gyrB* mutations, a mutation in a *RecA* gene, an insertion of a *lacI* gene expression cassette and/or a knockout of a *RecQ* gene may further include. The RecA gene in the mutated host cell expresses a protein with a decreased activity compared to the wild-type RecA gene (e.g., a recombination-deficient mutation), and the mutated RecA gene may be RecA1. The insertion site for the *lacI* gene expression cassette is at the yghX gene locus of the *Escherichia coli* host cell, and the *lacI* gene expression cassette includes a *lacIq* promoter, *lacI*, and/or a terminator. The genotype of the *Escherichia coli* host cell may be, for example, JM108[*gyrA*(H80A), *RecA1*, *lacIq*] and JM108[*gyrA*(H80A), *RecA1*, *lacIq*, *RecQ*].

Specifically, i) a vector comprising a nucleic acid molecule containing a poly(A) sequence may be introduced into an *Escherichia coli* host cell, and ii) the *Escherichia coli* host cell may be cultured under a condition that facilitates replication of the vector.

Alternatively, i) a vector comprising a nucleic acid molecule containing a poly(A) sequence may be introduced into an *Escherichia coli* host cell, ii) optionally, the *Escherichia coli* host cell may be cultured under a condition that facilitates replication of the vector, and iii) the *Escherichia coli* host cell may be cultured under a condition that facilitates expression of the vector, or the vector may be extracted from the *Escherichia coli* host cell of step i) or ii) and the vector may be transcribed in vitro.

The vector may be any suitable vector, such as a plasmid vector, a recombinant adenoviral vector, a recombinant lentiviral vector, and the like. For the construction of vectors, the replication conditions and specific operations for various vectors, and the expression conditions and specific operations may be performed by those skilled in the art in accordance with the actual situation and needs.

Alternatively, i) a nucleic acid molecule containing a poly(A) sequence may be integrated into the genome of an *Escherichia coli* host cell, and ii) the *Escherichia coli* host cell may be cultured under a condition that facilitates expression of the nucleic acid molecule containing the poly(A) sequence.

Those skilled in the art may integrate a nucleic acid molecule containing a poly(A) sequence into the genome of an *Escherichia coli* host cell, for example, by means of homologous recombination, and enable the expression of the nucleic acid molecule containing the poly(A) sequence. Those skilled in the art may find out the conditions that facilitate expression of the nucleic acid molecule containing the poly(A) sequence in accordance with the actual situation.

A nucleic acid molecule containing a poly(A) sequence may encode an mRNA with a poly(A) tail.

The poly(A) sequence may be any poly(A) sequence as long as it comprises consecutive repeating A bases. In some embodiments, the poly(A) sequence may comprise one single consecutive A base stretch. In some embodiments, the consecutive A base stretch may comprise 20-250 consecutive A bases. In some embodiments, the poly(A) sequence may comprise multiple (e.g., 2-5) consecutive A base stretches separated by a non-A base, wherein each consecutive A base stretch may comprise 10-100 consecutive A bases separated from each other by 1-20 non-A bases.

The inventors of the present application tested the replication of a variety of poly(A) sequences in recombinant host cells engineered to comprise a down-regulated gyrase activity, or a down-regulated gyrase expression levels, including consecutive poly(A) sequences, such as 80A, 100A, and 120A, and segmented poly(A) sequences, such as 30A+70A, and 30A+30A+43A. The results show that the host cells of the present application may improve the replication stability for various poly(A) sequences, and the effect is particularly pronounced on the highly unstable 120 As.

The present application further relates to the use of a host cell with a down-regulated gyrase activity or a down-regulated gyrase expression level, including a natural mutant strain and a recombinant strain, in the replication or expression of a nucleic acid molecule containing a poly(A) sequence.

A recombinant host cell engineered to comprise a down-regulated gyrase activity or a down-regulated gyrase expression level, whether obtained by the methods of the present application or by any other methods, including naturally occurring host cells in nature, are all within the scope of protection of the present application.

Hereinafter, the content of the present application will be further described in detail with reference to specific examples. The examples are given by way of example only, but do not limit the scope of protection of the present application.

### Example 1. Construction of strain mutants

CRISPR-Cas9 technology was used to mutate the *gyrA* gene and/or *gyrB* gene in the genomes of JM108, NEB Stable, Top10, DH5α or DH10B strains, wherein the sequences of the wild-type *gyrA* and *gyrB* genes prior to mutation are set forth in SEQ ID NO. 1 and SEQ ID NO. 2, respectively.

Specifically, in the JM108 strain, the *gyrA* gene was mutated to produce an expression product with either the H80A mutation or the A569T&T586A mutations. In addition, the *gyrB* gene was mutated to produce an expression product with the E42D mutation, the R136C mutation, or the D498A mutation.

1.1.1 SEQ ID NO. 1 and SEQ ID NO. 2 were uploaded to the sgRNA design website (www.atum.bio/eCommerce/cas9/input), to design 20 bp-long sgRNAs targeting a fragment of *gyrA* containing H80, a fragment of *gyrA* containing A569&T586, a fragment of *gyrB* containing E42, a fragment of *gyrB* containing R136, and a fragment of *gyrB* containing D498, respectively, as set forth in SEQ ID NOs. 8-12.

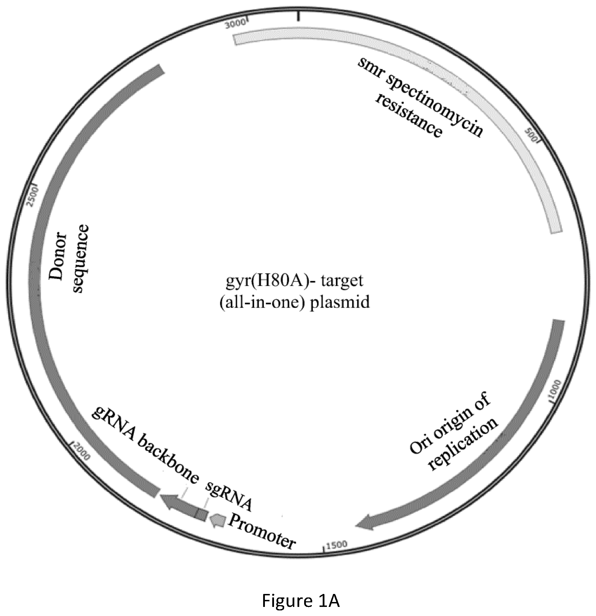

1.1.2 The aforementioned sgRNAs and their corresponding donor sequences (SEQ ID NOs. 13-17) were constructed on a target (all-in-one) plasmid, and the plasmid map is shown in FIG. 1A.

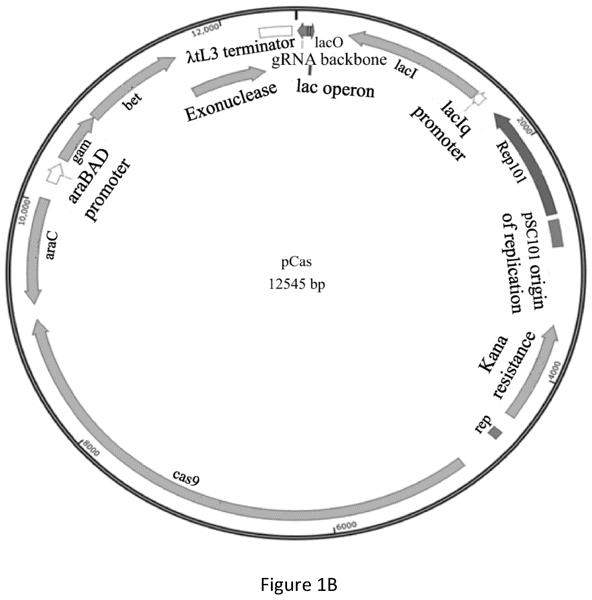

1.1.3 The pCas plasmid (plasmid map as shown in FIG. 1B) was transformed into chemically competent JM108, NEB Stable, Top10, DH5α or DH10B by means of chemical transformation, the transformed cells were spread onto an LB solid agar plate containing kanamycin, and incubated at 30°C for 14 hours with the plate inverted. Single colonies were selected and subjected to colony PCR using primers as set forth in SEQ ID NOs. 18 and 19, followed by Sanger sequencing to confirm positive clones.

**Table 1. Colony PCR primer sequence**

| SEQ I̅D̅ N̅O̅.̅ | P̅r̅i̅m̅e̅r̅ I̅D̅ | Sequence |
|---|---|---|
| SEQ I̅D̅ N̅O̅.̅ 1̅8̅ | p̅C̅a̅s̅-̅F̅ | GCATGACACCGGACATTATCCTGCAGC |
| SEQ ID NO. 19 | pCas-R | TCCCCAAATACAAAACCAATTTCAGCC |
| SEQ ID NO. 20 | *gyrA* (H80A)-F | CATCATACTGCCCGGCATGTTTTGC |
| SEQ ID NO. 21 | *gyrA*(H80A)-R | CGGCATCGGTGATCATCATGATCTGG |
| SEQ ID NO. 22 | *gyrA* (A569T&T586A)-F | CTCGCGATCGTGCTCATATCCTTGAAG |
| SEQ ID NO. 23 | *gyrA* (A569T&T586A)-R | ACACGTTGCAGACCCACTACGTTTTCA |
| SEQ ID NO. 24 | *gyrB* (E42D)-F | GATTTTTCACCGCCTGGAGCAATCTCAAG |
| SEQ ID NO. 25 | *gyrB* (E42D)-R | AAGCGTTACGTGTTGACCCAAAAAGTATAGAT |
| SEQ ID NO. 26 | *gyrB* (R136C)-F | TAGATGATTTTGCCTCTGAGCTTGATGATGAG |
| SEQ ID NO. 27 | *gyrB* (R136C)-R | AAGCGTTACGTGTTGACCCAAAAAGTATAGAT |
| SEQ ID NO. 28 | *gyrB* (D498A)-F | TAGATGATTTTGCCTCTGAGCTTGATGATGAG |
| SEQ ID NO. 29 | *gyrB* (D498A)-R | AAGCGTTACGTGTTGACCCAAAAAGTATAGAT |

1.1.4 The target plasmid was electroporated into the aforementioned five types of competent cells containing the pCas plasmid. The cells were spread onto a LB solid agar plate containing both kanamycin and spectinomycin and incubated at 30°C for 14 hours with the plate inverted. Single colonies were picked and subjected to colony PCR followed by Sanger sequencing to identify positive clones using primers SEQ ID NOs. 20-29, thereby obtaining successfully edited positive clones.

1.1.5 In the successfully edited positive clones obtained, 0.5 mM IPTG inducer was added to a 4 mL LB test tube containing positive clone bacterial culture and was incubated at 30°C for 7-8 hours, to eliminate the spectinomycin-resistant target plasmid.

1.1.6 A 4 mL LB test tube containing positive clone bacterial culture was incubated at 37°C for 7-8 hours to eliminate the kanamycin-resistant pCas plasmid.

1.1.7 The obtained strains were prepared into competent cells for later use.

When preparing a strain containing a combination of two of the above gene mutations, first the pCas plasmid was introduced through the above 1.1.3, then the target plasmid containing a sgRNA and a donor sequence corresponding to one mutation was introduced through the above 1.1.4, a successfully edited positive clone strain was obtained via screening, the target plasmid was removed from the strain through the above 1.1.5, and the strain was prepared into competent cells. After that, return to 1.1.4, the target plasmid containing a sgRNA and a donor sequence corresponding to another mutation was introduced, a successfully edited positive clone strain was obtained via screening, followed by proceeding in the order of 1.1.5-1.1.7. Specifically, when preparing a strain comprising the *gyrA* (H80A) and the *gyrB* (R136C) mutations, first the target plasmid containing a sgRNA and a donor sequence corresponding to *gyrA*(H80A) was introduced in 1.1.4, the target plasmid was removed from the strain via 1.1.5, the strain was prepared into competent cells. Subsequently, returning to 1.1.4, the target plasmid containing a sgRNA and a donor sequence corresponding to *gyrB* (R136C) was introduced.

Similarly, when preparing a strain containing combinations of three or more of the above-mentioned gene mutations, the operation was performed in the sequence of 1.1.3-1.1.4-1.1.5-(1.1.4-1.1.5)ₙ-1.1.6-1.1.7, wherein, n depends on the types of the aforementioned gene mutations to be included, and n is greater than or equal to 2.

The sequences of the edited *gyrA* gene were as set forth in SEQ ID NO. 3 and SEQ ID NO.4, (corresponding to H80A and A569T&T586A, respectively). The sequences of the edited *gyrB* gene were as set forth in SEQ ID NOs. 5-7 (corresponding to E42D, R136C, and D498A mutations, respectively).

### Example 2. Preparation of a plasmid containing a poly(A) sequence

Five test plasmids containing consecutive poly(A) sequences (SEQ ID NOs. 30-32) or segmented poly(A) sequences (SEQ ID NOs. 33 and SEQ ID NO. 34), namely poly(A)-Test 1 (80A), poly(A)-Test 2 (100A), poly(A)-Test 3 (120A), poly(A)-Test 4 (30&70A), and poly(A)-Test 5 (30&30&43A) were prepared.

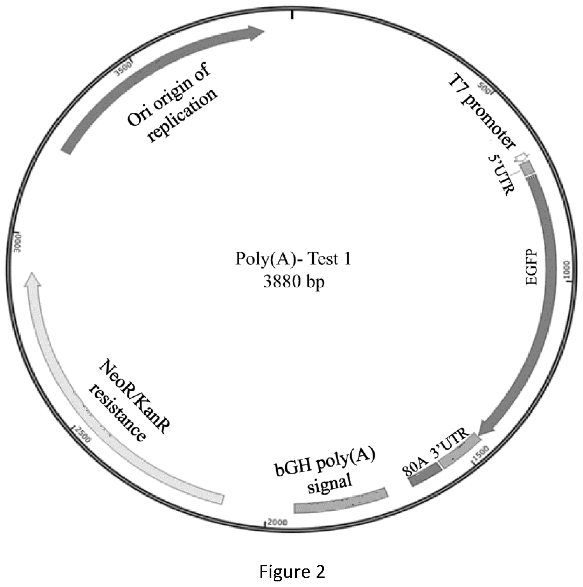

The map of the poly(A)-Test 1 (80A) plasmid is shown in FIG. 2, wherein the sequence encoding the mRNA portion is shown in SEQ ID NO. 35. Except for the differences in poly(A) sequences, the maps of the other test plasmids were identical to FIG. 2, and the sequence encoding the mRNA portion was identical to SEQ ID NO. 35 except for the differences in poly(A) sequences.

Specifically, the nucleotide sequences such as SEQ ID NO: 35, which sequentially contains a T7 promoter, a sequence encoding 5' UTR, a sequence encoding EGFP, a sequence encoding 3' UTR, a poly(A) sequence, and a linearization site BspQI, were synthesized (GenScript Biotech Corporation) and separately assembled into the kanamycin-resistant vector pVAX1 (GenScript Biotech Corporation, SEQ ID NO: 36) using the Gibson assembly method.

Positive clones were picked for Sanger sequencing, to obtain five test plasmids with correct sequencing.

### Example 3. Escherichia coli with mutated gyrA gene enhanced the replication stability of poly(A)

From the five test plasmids obtained in Example 2, 1 plasmid was selected at random, and transformed into the competent strains with mutated *gyrA* gene obtained in Example 1 and the commercial competent strains, including NEB Stable. 8 clones were picked from each transformed plate, cultured overnight using LB liquid medium at 37°C with shaking at 220 rpm. This resulting bacterium culture was designated as the first generation. The plasmids were extracted from the first-generation bacterial culture. Meanwhile, the first-generation bacterial culture was used as the seed culture for overnight cultivation at a 1:1000 inoculation ratio. This resulting bacterium culture was designated as the second generation, and serial passaging was continued until the third generation.

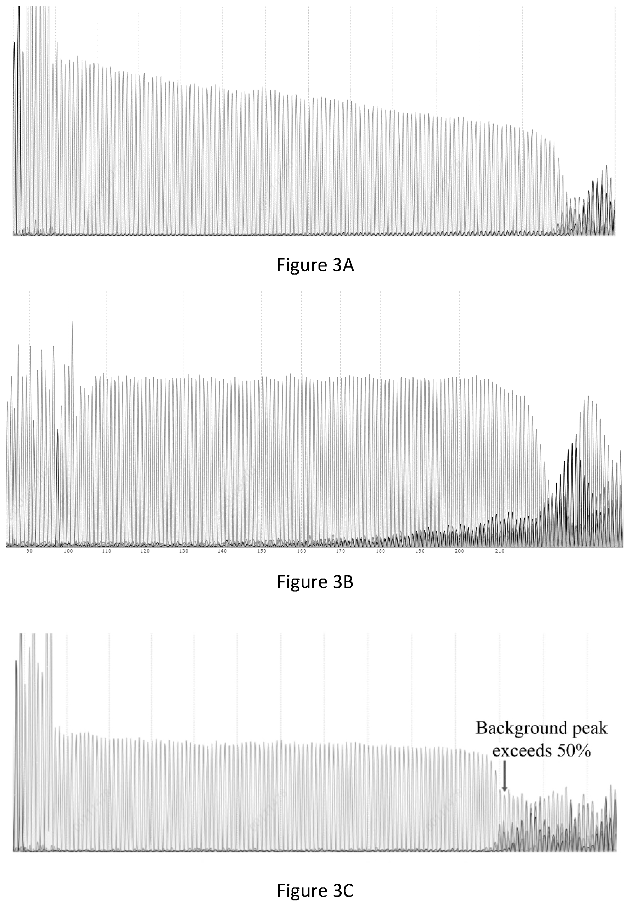

Plasmids extracted from the first- and third-generation passaged clones were selected, and Sanger sequencing was employed to verify the number of effective bases and purity qualification rate of the poly(A) sequence. The number of effective bases of poly(A) was defined as the number of bases between the first and last A bases in the poly(A) sequence where the background peak does not exceed 50%. A poly(A) sequence was recorded as purity-qualified when its number of effective bases deviated by no more than 1 nt from the initial number of bases of the transformed plasmid, the background peaks of the last three effective A bases were below 50%, and the background peak of the remaining poly(A) sequence was below 10%; otherwise, it was recorded as unqualified. The purity qualification rate was calculated on the basis of this. FIGs. 3A-3C show exemplary Sanger sequencing chromatograms of a poly(A) sequence with qualified purity (3A), a poly(A) sequence with unqualified purity (3B), and poly(A) deletion (3C), respectively.

On the basis of the data of number of effective bases and purity qualification rate of poly(A) of these passaged clones, the stability of poly(A) structure replication in different mutant host strains was evaluated. The higher the number of effective bases of poly(A) by Sanger sequencing, the higher the purity qualification rate of poly(A), indicating the greater the stability of poly(A) structure replication in the mutant host.

Table 2 shows the poly(A) effective bases of the poly(A)-Test 3 plasmids in the first- and third-generation strains, and the average purity qualification rate of the 5 test plasmids in the first- and third-generation strains with mutated *gyrA* gene.

The Sanger sequencing showed that in the first-generation NEB Stable strain, the number of effective bases of poly(A) was 115.6 nt for the poly(A)-Test 3 plasmid and the average purity qualification rate of poly(A) was 62.5% for the 5 test plasmids. In the third-generation clones, the number of effective bases of poly(A) was 114.8 nt for the poly(A)-Test 3 plasmid and the average purity qualification rate of poly(A) was 47.5% for the 5 test plasmids. However, in the NEB Stable strain with mutated *gyrA* gene, the corresponding number of effective bases and purity qualification rate of poly(A) were higher than those of commercial strains prior to engineering, no matter in the first generation or third generation. In the first-generation NEB Stable [*gyrA*(H80A)] and NEB Stable [*gyrA*(A569T, T586A)], the number of effective bases of poly(A) was 117.6 nt and 116.3 nt for the poly(A)-Test 3 plasmid and the average purity qualification rate of poly(A) was 80.0% and 67.5% for the 5 test plasmids. In the third-generation clones, the number of effective bases of poly(A) was 117.6 nt and 116.1 nt for the poly(A)-Test 3 plasmid and the average purity qualification rate of poly(A) was 72.5% and 65.0% for the 5 test plasmids. Similarly, in JM108, TOP10, DH5α and DH10B with mutated *gyrA* gene, the corresponding number of effective bases and purity qualification rate of poly(A) were higher compared to their respective unmutated commercial strains, no matter in the first generation or third generation.

The above data show that, *Escherichia coli* with mutated *gyrA* gene has a positive effect on the replication stability of poly(A) plasmids.

**Table 2. Passage stability of poly(A) plasmids in commercial strains and strains with mutated gyrA gene**

| Strain ID | First generation | | Third generation | |
|---|---|---|---|---|
| | Number of effective bases of poly(A) | Purity qualification rate of poly(A) | Number of effective bases of poly(A) | Purity qualification rate of poly(A) |
| JM108 | 116.9 | 55.0% | 116.5 | 47.5% |
| JM108 [*gyrA*(H80A)] | 118.3 | 82.5% | 118.1 | 77.5% |
| JM108 [*gyrA*(A569T, T586A)] | 117.4 | 75.0% | 116.9 | 65.0% |
| NEB Stable | 115.6 | 62.5% | 114.8 | 47.5% |
| NEB Stable [*gyrA*(H80A)] | 117.6 | 80.0% | 117.6 | 72.5% |
| NEB Stable [*gyrA*(A569T, T586A)] | 116.3 | 67.5% | 116.1 | 65.0% |
| TOP 10 | 115.5 | 55.5% | 115.8 | 40.0% |
| TOP 10 [*gyrA*(H80A)] | 116.7 | 62.5% | 117.6 | 57.5% |
| TOP 10 [*gyrA*(A569T, T586A)] | 116.6 | 65.0% | 115.9 | 57.5% |
| DH5α | 115.3 | 42.5% | 114.7 | 30.0% |
| DH5α [*gyrA*(H80A)] | 118.2 | 70.0% | 117.9 | 52.5% |
| DH5α [*gyrA*(A569T, T586A)] | 116.1 | 67.5% | 115.3 | 47.5% |
| DH10B | 115.9 | 52.5% | 115.7 | 45.0% |
| DH10B [*gyrA*(H80A)] | 117.5 | 72.5% | 117.3 | 65.0% |
| DH10B [*gyrA*(A569T, T586A)] | 117.3 | 75.0% | 116.9 | 57.5% |

### Example 4. Escherichia coli with mutated gyrB gene enhanced the replication stability of poly(A)

According to the operation of Example 3, from the five test plasmids obtained in Example 2, 1 plasmid was selected at random, and transformed into the competent -strains with mutated *gyrB* gene obtained in Example 1 and the commercial competent strains, including NEB Stable. The number of effective bases and purity qualification rate of poly(A) sequences in the first- and third-generation passaged clones of these strains were statistically analysed.

Table 3 summarizes the number of poly(A) effective bases of the poly(A)-Test 3 plasmids in the first- and third-generation strains, and the average purity qualification rate of poly(A) of the 5 test plasmids in the first- and third-generation strains with mutated *gyrB* gene.

The Sanger sequencing showed that in the first-generation NEB Stable strain, the number of effective bases of poly(A) was 115.6 nt for the poly(A)-Test 3 plasmid and the average purity qualification rate of poly(A) was 62.5% for the 5 test plasmids. In the third-generation clones, the number of effective bases of poly(A) was 114.8 nt for the poly(A)-Test 3 plasmid and the average purity qualification rate of poly(A) was 47.5% for the 5 test plasmids. However, in the NEB Stable strain with mutated *gyrB* gene, the corresponding number of effective bases and purity qualification rate of poly(A) for plasmids in either the first- or third-generation clones were higher than those of commercial strains prior to engineering. In NEB Stable[*gyrB*(E42D)], NEB Stable[*gyrB*(R136C)], and NEB Stable[*gyrB*(D498A)], the number of effective bases of poly(A) for the poly(A)-Test 3 plasmid in the first-generation clones was 116.2 nt, 117.6 nt and 116.9 nt, respectively, and the average purity qualification rate of poly(A) for the 5 test plasmids was 67.5%, 77.5% and 72.5%, respectively. In the third-generation clone, the number of effective bases of poly(A) for the poly(A)-Test 3 plasmid was 115.2 nt, 117.1 nt and 116.3 nt, respectively, and the average purity qualification rate of poly(A) for the 5 test plasmids was 57.5%, 67.5% and 60.0%, respectively. Similarly, in JM108, TOP10, DH5α and DH10B with mutated *gyrB* gene, the corresponding number of effective bases and/or purity qualification rate of poly(A), especially the purity qualification rate, were higher compared to their respective unmutated commercial strains, no matter in the first generation or third generation.

The above data show that, *Escherichia coli* with mutated *gyrB* gene has a positive effect on the replication stability of poly(A) plasmids.

**Table 3. Passage stability of poly(A) plasmids in commercial strains and strains with mutated gyrB gene**

| Strain ID | First generation | | Third generation | |
|---|---|---|---|---|
| | Number of effective bases of poly(A) | Purity qualification rate of poly(A) | Number of effective bases of poly(A) | Purity qualification rate of poly(A) |
| JM108 | 116.9 | 55.0% | 115.5 | 47.5% |
| JM108 [*gyrB*(E42D)] | 116.7 | 70.0% | 115.9 | 57.5% |
| JM108 [*gyrB*(R136C)] | 118.1 | 80.0% | 117.3 | 67.5% |
| JM108 [*gyrB*(D498A)] | 116.9 | 72.5% | 116.6 | 60.0% |
| NEB Stable | 115.6 | 62.5% | 114.8 | 47.5% |
| NEB Stable [*gyrB*(E42D)] | 116.2 | 67.5% | 115.2 | 57.5% |
| NEB Stable [*gyrB*(R136C)] | 117.6 | 77.5% | 117.1 | 67.5% |
| NEB Stable [*gyrB*(D498A)] | 116.9 | 72.5% | 116.3 | 60.0% |
| TOP 10 | 115.5 | 55.5% | 115.8 | 40.0% |
| TOP 10 [*gyrB*(E42D)] | 115.4 | 60.0% | 115.6 | 52.5% |
| TOP 10 [*gyrB*(R136C)] | 116.1 | 70.0% | 116.2 | 60.0% |
| TOP 10 [*gyrB*(D498A)] | 116.9 | 67.5% | 115.3 | 55.0% |
| DH5α | 115.3 | 42.5% | 114.7 | 30.0% |
| DH5α [*gyrB*(E42D)] | 117.1 | 70.0% | 116.6 | 62.5% |
| DH5α [*gyrB*(R136C)] | 117.0 | 70.0% | 116.8 | 65.0% |
| DH5α [*gyrB*(D498A)] | 116.1 | 62.5% | 115.6 | 52.5% |
| DH10B | 115.9 | 52.5% | 115.7 | 45.0% |
| DH10B [*gyrB*(E42D)] | 116.8 | 70.0% | 116.3 | 57.5% |
| DH10B [*gyrB*(R136C)] | 117.3 | 72.5% | 117.6 | 65.0% |
| DH10B [*gyrB*(D498A)] | 117.1 | 72.5% | 116.4 | 62.5% |

### Example 5. Escherichia coli with mutated gyrA & gyrB genes enhanced the replication stability of poly(A)

According to the operation of Example 3, from the five test plasmids obtained in Example 2, 1 plasmid was selected at random, and transformed into the competent strains with mutated *gyrA*&*gyrB* genes obtained in Example 1 and the commercial competent strains, including NEB Stable. The number of effective bases and purity qualification rate of poly(A) sequences in the first- and third-generation passaged clones were statistically analysed.

Table 4 summarizes the number of effective bases of poly(A) for the poly(A)-Test 3 plasmids in the first- and third-generation strains, and the purity qualification rate of poly(A) for the five test plasmids in the first- and third-generation strains.

The Sanger sequencing showed that in the first-generation NEB Stable strain, the number of effective bases of poly(A) was 115.6 nt for the poly(A)-Test 3 plasmid and the average purity qualification rate of poly(A) was 62.5% for the 5 test plasmids. In the third-generation clones, the number of effective bases of poly(A) was 114.8 nt for the poly(A)-Test 3 plasmid and the average purity qualification rate of poly(A) was 47.5% for the 5 test plasmids. In the NEB Stable strain with simultaneous mutations in both the *gyrA* gene and *gyrB* gene, the corresponding number of effective bases and purity qualification rate of poly(A) were higher than those of commercial strains prior to engineering, no matter in the first generation or third generation. In the NEB Stable [*gyrA*(H80A) *gyrB*(R136C)], the number of effective bases of poly(A) was 118.1 nt for the poly(A)-Test 3 plasmid in the first-generation passaged clones and the average purity qualification rate of poly(A) was 87.5% for the 5 test plasmids. In the third-generation clones, the number of effective bases of poly(A) was 117.9 nt for the poly(A)-Test 3 plasmid and the average purity qualification rate of poly(A) was 75.0% for the 5 test plasmids. Similarly, in JM108, TOP10, DH5α and DH10B with mutated *gyrA&gyrB* genes, the corresponding number of effective bases and purity qualification rate of poly(A) were higher compared to their respective unmutated commercial strains, no matter in the first generation or third generation.

The above data show that, *Escherichia coli* with simultaneous mutations in the *gyrA* gene and *gyrB* gene has a positive effect on the replication stability of the poly(A) plasmids.

**Table 4. Passage stability of poly(A) plasmids in hosts with mutated gyrA gene and gyrB gene and commercial strains**

| Strain ID | First generation | | Third generation | |
|---|---|---|---|---|
| | Number of effective bases of poly(A) | Purity qualification rate of poly(A) | Number of effective bases of poly(A) | Purity qualification rate of poly(A) |
| JM108 | 116.9 | 55.0% | 116.5 | 47.5% |
| JM108 [*gyrA* (H80A) *gyrB* (R136C)] | 118.2 | 82.5% | 117.7 | 77.5% |
| NEB Stable | 115.6 | 62.5% | 114.8 | 47.5% |
| NEB Stable [*gyrA* (H80A) *gyrB* (R136C)] | 118.1 | 87.5% | 117.9 | 75.0% |
| TOP 10 | 115.5 | 55.5% | 115.8 | 40.0% |
| TOP 10 [*gyrA* (H80A) *gyrB* (R136C)] | 116.9 | 67.5% | 116.6 | 62.5% |
| DH5α | 115.3 | 42.5% | 114.7 | 30.0% |
| DH5α[*gyrA* (H80A) *gyrB* (R136C)] | 117.4 | 70.0% | 117.1 | 52.5% |
| DH10B | 115.9 | 52.5% | 115.7 | 45.0% |
| DH10B [*gyrA* (H80A) *gyrB* (R136C)] | 117.2 | 77.5% | 116.8 | 67.5% |

### Example 6. Plasmid output in mutant strains

The poly(A)-Test 3 plasmid obtained in Example 2 was selected and transformed into a mutant strain with better poly(A) stability, namely JM108 [*gyrA*(H80A)], NEB Stable [*gyrA*(H80A)], DH10B [*gyrA*(H80A)], JM108 [*gyrB*(R136C)] and JM108 [*gyrA*(H80A) *gyrB*(R136C)], and the corresponding commercial strains JM108, NEB Stable, DH10B. A single colony was randomly selected and inoculated into 4 mL of LB liquid medium, followed by culturing at 30°C with shaking at 200 rpm for 15 hours to obtain a seed culture. The seed culture was inoculated into 100 mL of LB liquid medium at a ratio of 1 : 1000, cultured at 37°C with shaking at 200 rpm for 13 hours. Plasmids were then extracted, and the plasmid output was measured using a nanodrop spectrophotometer.

Table 5 summarizes the plasmid outputs from poly(A)-Test 3 plasmid transformation in different strains, showing that the plasmid outputs obtained from mutant strains were nearly identical to those from non-mutant strains.

**Table 5. Plasmid outputs of poly(A) plasmids in hosts with mutated gyrA gene and/or gyrB gene and commercial strains**

| Strain ID | Plasmid output (µg) |
|---|---|
| JM108 | 388.35 |
| JM108 [*gyrA* (H80A)] | 369.73 |
| JM108 [*gyrB* (R136C)] | 349.98 |
| JM108 [*gyrA* (H80A) *gyrB*(R136C)] | 403.12 |
| NEB Stable | 422.06 |
| NEB Stable [*gyrA* (H80A)] | 400.98 |
| DH10B | 350.57 |
| DH10B [*gyrA* (H80A)] | 322.97 |

### Example 7. Supercoil ratio of plasmids in mutant strains

The poly(A)-Test 2 (100A) plasmid and poly(A)-Test 3 (120A) plasmid obtained in Example 2 were selected and separately transformed into the commercial strain JM108 and the mutant strains JM108[*gyrA*(H80A)], JM108[*gyrB*(R136C)], and JM108[*gyrA*(H80A)*gyrB*(R136C)] following the procedures described in Example 3. Single colonies were randomly picked for serial passage experiments.

Plasmids were extracted from the first- and third-generation strains. 200 ng of the extracted plasmids were subjected to DNA gel electrophoresis, and the supercoil ratio of the plasmids was analysed by the fully automatic gel imaging and analysis system GIS300.

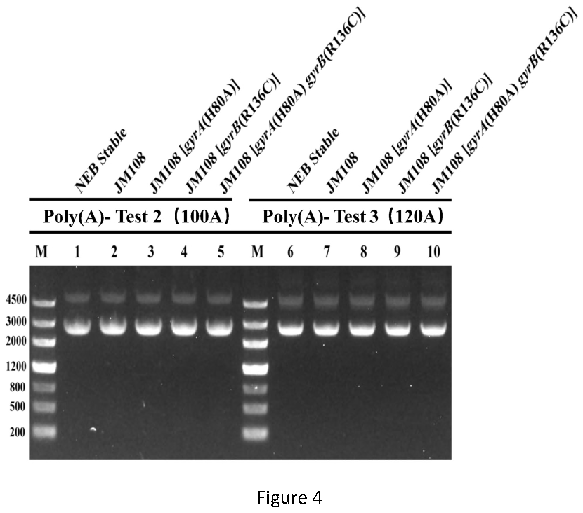

FIG. 4 shows the average supercoil ratio corresponding to each strain. It may be seen that the supercoil ratio of the plasmids obtained in the mutant strains was nearly identical to that of JM108, both of which were greater than 80%.

In summary, mutating the *gyrA* gene and/or *gyrB* gene enables *Escherichia coli* hosts to enhance the replication stability of poly(A) plasmids without affecting plasmid quality, such as the supercoil ratio.

### Example 8. Escherichia coli with gyrA mutation combined with other genotypic mutations further enhanced the replication stability of poly(A) sequences

An ATP-dependent DNA recombinase was expressed from the *RecA* gene, which assisted DNA in locating and pairing the correct sequences when it is damaged or needs to undergo replication, thereby facilitating the completion of DNA repair or replication. *RecA1* is a recombination-deficient mutant of the *RecA* gene, which may reduce the non-specific recombination of DNA, reduce the recombination probability of exogenous DNA, thereby making the inserted exogenous DNA more stable, and facilitating DNA transformation and the extraction of high-purity plasmids.

The Lac promoter is a common functional component in commercial vectors at present, often serving as the initiating element for blue-white screening, such as in pUC57, in which the multiple cloning site (MCS) is close to the Lac promoter. When researchers insert the mRNA functional sequence into the MCS, the polyA sequence often ends up downstream of the Lac promoter, and the transcriptional activity of the Lac promoter will lead to a decrease in the stability of the poly(A) sequence. *LacIq* is a variant of the *lacI* gene, which can express a large number of repressor proteins, thereby reducing the transcriptional activity of the Lac promoter under non-induced conditions and improving the stability of its downstream poly(A) sequence.

The *RecQ* gene belongs to *RecQ* helicase family and is involved in DNA unwinding, replication, DNA damage repair and other processes. Knockout of the *RecQ* gene makes the inserted exogenous DNA more stable, which is conducive to the efficient transformation of DNA.

Referring to the method of Example 1, CRISPR-Cas9 technology was used to mutate, knockout or knockin related genes on the genome of *Escherichia coli* JM108 strain. Wherein, the gene to be knockout was as follows: *RecQ*(SEQ ID NO. 41), the gene to be knocked in was as follows: lacIq promoter + lacI + rrnB T1 terminator + rrnB T2 terminator(SEQ ID NO. 42), the wild-type gene to be mutated was as follows: *RecA*(SEQ ID NO. 43). Specifically, wild-type *RecA* gene was mutated into *RecA1* (SEQ ID NO. 44).

Referring to the method of Example 1, the *Escherichia coli* mutant strain was constructed, and the corresponding competent gene mutant strains JM108[*gyrA*(H80A), *RecA1*, *lacIq*] and JM108[*gyrA*(H80A), *RecA1*, *lacIq*, *RecQ*], were prepared. The sgRNA sequences targeting *RecQ*, yghX (Gene ID:2847694) and *RecA* were as set forth in SEQ ID NOs. 45-47, the corresponding donor sequences were as set forth in SEQ ID NOs. 48-50, and the colony PCR primer sequences were listed in Table 6.

**Table 6. Colony PCR primer sequences**

| SEQ ID NO. | Primer ID | Sequence |
|---|---|---|
| S̅E̅Q̅ I̅D̅ N̅O̅.̅5̅1̅ | *̅R̅e̅c̅A̅-̅*̅F̅ | CATTGCAGACCTTGTGGCAACAAT |
| SEQ ID NO.52 | *RecA-*R | GGATGTTGATTCTGTCATGGCATATCCTTACAAC |
| SEQ ID NO.53 | *RecQ-*F | TATAACCACGACTCTAACGGGCGTTCC |
| SEQ ID NO.54 | *RecQ-*R | CGAAATAATCAAATGAATGCCAAATCCGGC |
| SEQ ID NO.55 | *lacI-*F | CATACTGCCCTTTGTACTTCTCGAGAGC |
| SEQ ID NO.56 | *lacI-*R | ACCCAGCGGTTCAGTAAATTGTACGA |

According to the operation of Example 3, 1 plasmid was randomly selected from each of the five test plasmids obtained in Example 2 and transformed into competent JM108[*gyrA*(H80A), *RecA1*, *lacIq*] and JM108[*gyrA*(H80A), *RecA1*, *lacIq*, *RecQ*] gene mutant strains obtained according to the method of Example 1, and the corresponding commercial strain JM108. The number of effective bases and purity qualification rate of poly(A) sequences in the first- and third-generation passaged clones were statistically analysed.

Table 7 summarizes the number of effective bases of poly(A) for the poly(A)-Test 3 plasmids in the first- and third-generation strains, and the purity qualification rate of poly(A) for the five test plasmids in the first- and third-generation strains.

The Sanger sequencing showed that in the first-generation JM108 strain, the number of effective bases of poly(A) was 116.9 nt for the poly(A)-Test 3 plasmid and the average purity qualification rate of poly(A) was 55.0% for the 5 test plasmids. In the third-generation clones, the number of effective bases of poly(A) was 116.5 nt for the poly(A)-Test 3 plasmid and the average purity qualification rate of poly(A) was 47.5% for the 5 test plasmids. However, in the mutant strains, the corresponding number of effective bases and purity qualification rate of poly(A) were higher than those of the commercial strain JM108, no matter in the first generation or third generation. In the first-generation JM108[*gyrA*(H80A), *RecA1*, *lacIq*] and JM108[*gyrA*(H80A), *RecA1*, *lacIq*, *RecQ*] mutant strains, the number of effective bases of poly(A) for poly (A)-Test 3 plasmid was 118.6 nt and 118.3 nt, respectively, and the average purity qualification rate of poly(A) for the 5 test plasmids were 83.2% and 85.5%, respectively. In the third-generation clones, the number of effective bases of poly(A) for poly (A)-Test 3 plasmid was 117.9 nt and 118.1 nt, respectively, and the average purity qualification rate of poly(A) for the 5 test plasmids were 80.5% and 82.5%, respectively.

The above data show that the combined mutation or knockout of *gyrA*(H80A) with genotypes such as *RecA1*, *lacIq* and *RecQ* may further enhance the replication stability of the poly(A) plasmid in the strain.

**Table 7. Replication stability of poly(A) plasmids in hosts with gyrA mutation combined with other genotypic mutations and in commercial strains**

| Strain ID | First generation | | Third generation | |
|---|---|---|---|---|
| | Number of effective bases of poly(A) | Purity qualification rate of poly(A) | Number of effective bases of poly(A) | Purity qualification rate of poly(A) |
| JM108 | 116.9 | 55.0% | 116.5 | 47.5% |
| JM108[*gyrA* (H80A)] | 118.3 | 82.5% | 118.1 | 77.5% |
| JM108[*gyrA* (H80A), *RecA1*, *lacIq*] | 118.6 | 83.2% | 117.9 | 80.5% |
| JM108 [*gyrA* (H80 A), *RecA1*, *lacIq*, *RecQ*] | 118.3 | 85.5% | 118.1 | 82.5% |

### Example 9. Escherichia coli with gyrA mutation combined with other genotypic mutations further enhanced the serial passage replication stability of poly(A) plasmids

The five test plasmids obtained in Example 2 were separately transformed into JM108[*gyrA*(H80A), *RecA1*, *lacIq*, *RecQ*] obtained in Example 1 and the commercial strain JM108. 16 clones were picked from each transformed plate, cultured overnight using LB liquid medium at 37°C with shaking at 220 rpm. This resulting bacterium culture was designated as the passage template (P0 generation). 200 µl of the P0-generation bacterial culture and 200 µl of 50% glycerol were mixed and stored at -80°C. Plasmids were extracted from the P0-generation bacterial culture, and Sanger sequencing was used to verify the number of effective bases and the peak-pattern purity of the poly(A) sequences. 4 clones that passed the Sanger sequencing results were selected. The corresponding P0-generation bacteria mixed with glycerol were used as the seed culture and inoculated at a ratio of 1 : 1000 and cultured overnight. This resulting bacterium culture was designated as the first generation (P1 generation). At the same time, the first-generation bacterial culture was used as the seed culture and inoculated at a ratio of 1 : 1000 and cultured overnight. This bacterium culture was designated as the second generation. Serial passaging was continued until the tenth generation (P10 generation).

The plasmids extracted from the first-generation (P1-generation) to the tenth-generation (P10-generation) passaged clones were selected, and Sanger sequencing was used to verify whether the number of effective bases and purity of poly(A) were qualified. When the number of effective bases and purity were qualified, the clone was recorded as qualified, otherwise the clone was recorded as unqualified, and the clone qualification rate was calculated accordingly. On the basis of the qualification rate data of passaged clones of each generation, the stability of poly(A) structure replication in different mutant host strains was evaluated. The higher the qualification rate of passaged clones of each generation, the higher the stability of poly(A) structure replication in the corresponding mutant host. Table 8 shows the poly(A) clone qualification rate for the 5 test plasmids in the first-generation (P1) to the tenth-generation (P10) strains.

The Sanger sequencing showed that in the first-generation JM108 strain, the average qualification rate of the 5 test plasmids of passaged clones was 60.7%. With the progress of passage, the qualification rate of passaged clones of JM108 strain decreased rapidly, and the average qualification rate of passaged clones of the fifth generation was lower than 42.0%, and the average qualification rate of passaged clones of the tenth generation was 14.6% only. However, in JM108[*gyrA*(H80A), *RecA1*, *lacIq*, *RecQ*] gene mutant strains, the qualification rate of corresponding poly(A) of passaged clones was higher than that of commercial strain JM108, no matter in the first generation or the tenth generation. In the first-generation JM108[*gyrA*(H80A), *RecA1*, *lacIq*, *RecQ*] gene mutant strains, the average qualification rate of the 5 test plasmids of passaged clones was 87.5%. The average qualification rate of passaged clones was still 42.0% when the strain was passaged to the tenth generation, and higher than the qualification rate of passaged clones of the fifth generation of commercial strain JM108.

The above data show that the combined mutation or knockout of *gyrA*(H80A) with genotypes such as *RecA1*, *lacIq* and *RecQ* exhibits a positive effect on the passage stability of poly(A) plasmids.

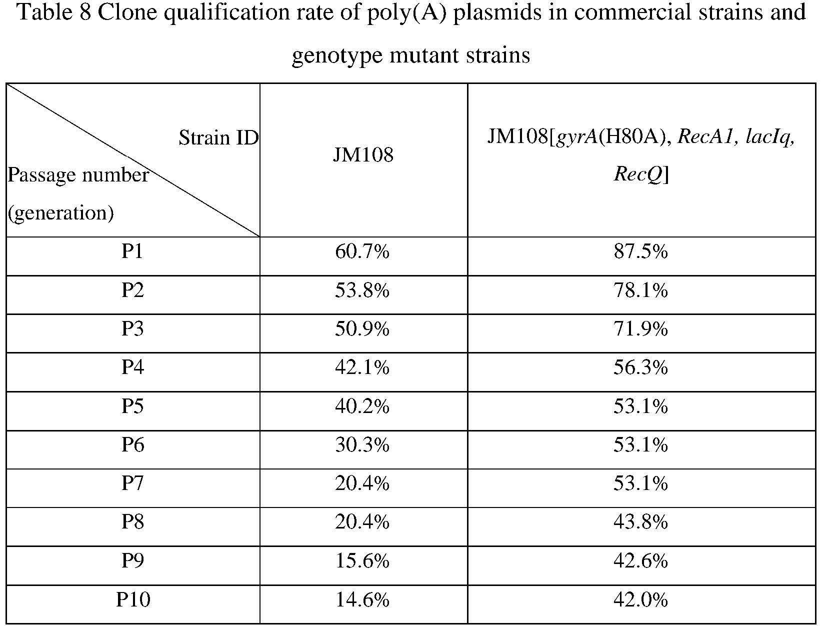

In particular, for plasmids with highly challenging poly(A) structures, such as those containing consecutive A bases longer than 120 bp, when using the strains described in the present application, the advantage in poly(A) replication stability becomes increasingly pronounced with serial passages, compared with commercial strains.

The embodiments of the present invention are not limited to the above examples, and various modifications and improvements in forms and details may be made to the present invention by those of ordinary skill in the art without departing from the spirit and scope of the present invention, and these modifications and improvements all fall within the scope of protection of the present invention.

The nucleotide sequences mentioned herein are as follows.
SEQ ID NO.1: Wild-type *gyrA* 
ATGAGCGACCTTGCGAGAGAAATTACACCGGTCAACATTGAGGAAGAGCTGAAGAGCTCCTA
TCTGGATTATGCGATGTCGGTCATTGTTGGCCGTGCGCTGCCAGATGTCCGAGATGGCCTGAA
GCCGGTACACCGTCGCGTACTTTACGCCATGAACGTACTAGGCAATGACTGGAACAAAGCCTA
TAAAAAATCTGCCCGTGTCGTTGGTGACGTAATCGGTAAATACCATCCCCATGGTGACTCGGC
GGTCTATGACACGATCGTCCGCATGGCGCAGCCATTCTCGCTGCGTTATATGCTGGTAGACGGT
CAGGGTAACTTCGGTTCTATCGACGGCGACTCTGCGGCGGCAATGCGTTATACGGAAATCCGT
CTGGCGAAAATTGCCCATGAACTGATGGCCGATCTCGAAAAAGAGACGGTCGATTTCGTTGAT
AACTATGACGGCACGGAAAAAATTCCGGACGTCATGCCAACCAAAATTCCTAACCTGCTGGT
GAACGGTTCTTCCGGTATCGCCGTAGGTATGGCAACCAACATCCCGCCGCACAACCTGACGG
AAGTCATCAACGGTTGTCTGGCGTATATTGATGATGAAGACATCAGCATTGAAGGGCTGATGG
AACACATCCCGGGGCCGGACTTCCCGACGGCGGCAATCATTAACGGTCGTCGCGGTATTGAA
GAAGCTTACCGTACCGGTCGCGGCAAGGTGTATATCCGCGCTCGCGCAGAAGTGGAAGTTGA
CGCCAAAACCGGTCGTGAAACCATTATCGTCCACGAAATTCCGTATCAGGTAAACAAAGCGC
GCCTGATCGAGAAGATTGCGGAACTGGTAAAAGAAAAACGCGTGGAAGGCATCAGCGCGCT
GCGTGACGAGTCTGACAAAGACGGTATGCGCATCGTGATTGAAGTGAAACGCGATGCGGTCG
GTGAAGTTGTGCTCAACAACCTCTACTCCCAGACCCAGTTGCAGGTTTCTTTCGGTATCAACA
TGGTGGCATTGCACCATGGTCAGCCGAAGATCATGAACCTGAAAGACATCATCGCGGCGTTTG
TTCGTCACCGCCGTGAAGTGGTGACCCGTCGTACTATTTTCGAACTGCGTAAAGCTCGCGATC
GTGCTCATATCCTTGAAGCATTAGCCGTGGCGCTGGCGAACATCGACCCGATCATCGAACTGA
TCCGTCATGCGCCGACGCCTGCAGAAGCGAAAACTGCGCTGGTTGCTAATCCGTGGCAGCTG
GGCAACGTTGCCGCGATGCTCGAACGTGCTGGCGACGATGCTGCGCGTCCGGAATGGCTGGA

GCCAGAGTTCGGCGTGCGTGATGGTCTGTACTACCTGACCGAACAGCAAGCTCAGGCGATTC
TGGATCTGCGTTTGCAGAAACTGACCGGTCTTGAGCACGAAAAACTGCTCGACGAATACAAA
GAGCTGCTGGATCAGATCGCGGAACTGTTGCGTATTCTTGGTAGCGCCGATCGTCTGATGGAA
GTGATCCGTGAAGAGCTGGAGCTGGTTCGTGAACAGTTCGGTGACAAACGTCGTACTGAAAT
CACCGCCAACAGCGCAGACATCAACCTGGAAGATCTGATCACCCAGGAAGATGTGGTCGTGA
CGCTCTCTCACCAGGGCTACGTTAAGTATCAGCCGCTTTCTGAATACGAAGCGCAGCGTCGTG
GCGGGAAAGGTAAATCTGCCGCACGTATTAAAGAAGAAGACTTTATCGACCGACTGCTGGTG
GCGAACACTCACGACCATATTCTGTGCTTCTCCAGCCGTGGTCGCGTCTATTCGATGAAAGTTT
ATCAGTTGCCGGAAGCCACTCGTGGCGCGCGCGGTCGTCCGATCGTCAACCTGCTGCCGCTG
GAGCAGGACGAACGTATCACTGCGATCCTGCCAGTGACCGAGTTTGAAGAAGGCGTGAAAG
TCTTCATGGCGACCGCTAACGGTACCGTGAAGAAAACTGTCCTCACCGAGTTCAACCGTCTG
CGTACCGCCGGTAAAGTGGCGATCAAACTGGTTGACGGCGATGAGCTGATCGGCGTTGACCT
GACCAGCGGCGAAGACGAAGTAATGCTGTTCTCCGCTGAAGGTAAAGTGGTGCGCTTTAAAG
AGTCTTCTGTCCGTGCGATGGGCTGCAACACCACCGGTGTTCGCGGTATTCGCTTAGGTGAAG
GCGATAAAGTCGTCTCTCTGATCGTGCCTCGTGGCGATGGCGCAATCCTCACCGCAACGCAAA
ACGGTTACGGTAAACGTACCGCAGTGGCGGAATACCCAACCAAGTCGCGTGCGACGAAAGG
GGTTATCTCCATCAAGGTTACCGAACGTAACGGTTTAGTTGTTGGCGCGGTACAGGTAGATGA
CTGCGACCAGATCATGATGATCACCGATGCCGGTACGCTGGTACGTACTCGCGTTTCGGAAAT
CAGCATCGTGGGCCGTAACACCCAGGGCGTGATCCTCATCCGTACTGCGGAAGATGAAAACG
TAGTGGGTCTGCAACGTGTTGCTGAACCGGTTGACGAGGAAGATCTGGATACCATCGACGGC
AGTGCCGCGGAAGGGGACGATGAAATCGCTCCGGAAGTGGACGTTGACGACGAGCCAGAAG
AAGAATAA
SEQ ID NO. 37: Wild-type GyrA 
MSDLAREITPVNIEEELKSSYLDYAMSVIVGRALPDVRDGLKPVHRRVLYAMNVLGNDWNKAYK
KSARVVGDVIGKYHPHGDSAVYDTIVRMAQPFSLRYMLVDGQGNFGSIDGDSAAAMRYTEIRLA
KIAHELMADLEKETVDFVDNYDGTEKIPDVMPTKIPNLLVNGSSGIAVGMATNIPPHNLTEVINGC
LAYIDDEDISIEGLMEHIPGPDFPTAAIINGRRGIEEAYRTGRGKVYIRARAEVEVDAKTGRETIIVH
EIPYQVNKARLIEKIAELVKEKRVEGISALRDESDKDGMRIVIEVKRDAVGEVVLNNLYSQTQLQV
SFGINMVALHHGQPKIMNLKDIIAAFVRHRREVVTRRTIFELRKARDRAHILEALAVALANIDPIIE
LIRHAPTPAEAKTALVANPWQLGNVAAMLERAGDDAARPEWLEPEFGVRDGLYYLTEQQAQAIL
DLRLQKLTGLEHEKLLDEYKELLDQIAELLRILGSADRLMEVIREELELVREQFGDKRRTEITANS
ADINLEDLITQEDVVVTLSHQGYVKYQPLSEYEAQRRGGKGKSAARIKEEDFIDRLLVANTHDHI
LCFSSRGRVYSMKVYQLPEATRGARGRPIVNLLPLEQDERITAILPVTEFEEGVKVFMATANGTVK
KTVLTEFNRLRTAGKVAIKLVDGDELIGVDLTSGEDEVMLFSAEGKVVRFKESSVRAMGCNTTGV
RGIRLGEGDKVVSLIVPRGDGAILTATQNGYGKRTAVAEYPTKSRATKGVISIKVTERNGLVVGAV
QVDDCDQIMMITDAGTLVRTRVSEISIVGRNTQGVILIRTAEDENVVGLQRVAEPVDEEDLDTIDG
SAAEGDDEIAPEVDVDDEPEEE*
SEQ ID NO. 38: GyrA mutant 
MSDLAREITPVNIEEELKSSYLDYAMSVIVGRALPDVRDGLKPVHRRVLYAMNVLGNDWNKAYK
KSARVVGDVIGKYHPX1GDSAVYDTIVRMAQPFSLRYMLVDGQGNFGSIDGDSAAAMRYTEIRL
AKIAHELMADLEKETVDFVDNYDGTEKIPDVMPTKIPNLLVNGSSGIAVGMATNIPPHNLTEVING
CLAYIDDEDISIEGLMEHIPGPDFPTAAIINGRRGIEEAYRTGRGKVYIRARAEVEVDAKTGRETIIV
HEIPYQVNKARLIEKIAELVKEKRVEGISALRDESDKDGMRIVIEVKRDAVGEVVLNNLYSQTQLQ
VSFGINMVALHHGQPKIMNLKDIIAAFVRHRREVVTRRTIFELRKARDRAHILEALAVALANIDPII
ELIRHAPTPAEAKTALVANPWQLGNVAAMLERAGDDAARPEWLEPEFGVRDGLYYLTEQQAQAI
LDLRLQKLTGLEHEKLLDEYKELLDQIAELLRILGSADRLMEVIREELELVREQFGDKRRTEITAN
SADINLEDLITQEDVVVTLSHQGYVKYQPLSEYEAQRRGGKGKSX2ARIKEEDFIDRLLVANX3H

DHILCFSSRGRVYSMKVYQLPEATRGARGRPIVNLLPLEQDERITAILPVTEFEEGVKVFMATANG
TVKKTVLTEFNRLRTAGKVAIKLVDGDELIGVDLTSGEDEVMLFSAEGKVVRFKESSVRAMGCNT
TGVRGIRLGEGDKVVSLIVPRGDGAILTATQNGYGKRTAVAEYPTKSRATKGVISIKVTERNGLVV
GAVQVDDCDQIMMITDAGTLVRTRVSEISIVGRNTQGVILIRTAEDENVVGLQRVAEPVDEEDLDT
IDGSAAEGDDEIAPEVDVDDEPEEE*
 X1 = H or A, X2= A or T, X3 = T or A
SEQ ID NO. 2: Wild-type *gyrB* 
ATGTCGAATTCTTATGACTCCTCCAGTATCAAAGTCCTGAAAGGGCTGGATGCGGTGCGTAAG
CGCCCGGGTATGTATATCGGCGACACGGATGACGGCACCGGTCTGCACCACATGGTATTCGAG
GTGGTAGATAACGCTATCGACGAAGCGCTCGCGGGTCACTGTAAAGAAATTATCGTCACCATT
CACGCCGATAACTCTGTCTCTGTACAGGATGACGGGCGCGGCATTCCGACCGGTATTCACCCG
GAAGAGGGCGTATCGGCGGCGGAAGTGATCATGACCGTTCTGCACGCAGGCGGTAAATTTGA
CGATAACTCCTATAAAGTGTCCGGCGGTCTGCACGGCGTTGGTGTTTCGGTAGTAAACGCCCT
GTCGCAAAAACTGGAGCTGGTTATCCAGCGCGAGGGTAAAATTCACCGTCAGATCTACGAAC
ACGGTGTACCGCAGGCCCCGCTGGCGGTTACCGGCGAGACTGAAAAAACCGGCACCATGGT
GCGTTTCTGGCCCAGCCTCGAAACCTTCACCAATGTGACCGAGTTCGAATATGAAATTCTGGC
GAAACGTCTGCGTGAGTTGTCGTTCCTCAACTCCGGCGTTTCCATTCGTCTGCGCGACAAGCG
CGACGGCAAAGAAGACCACTTCCACTATGAAGGCGGCATCAAGGCGTTCGTTGAATATCTGA
ACAAGAACAAAACGCCGATCCACCCGAATATCTTCTACTTCTCCACTGAAAAAGACGGTATTG
GCGTCGAAGTGGCGTTGCAGTGGAACGATGGCTTCCAGGAAAACATCTACTGCTTTACCAAC
AACATTCCGCAGCGTGACGGCGGTACTCACCTGGCAGGCTTCCGTGCGGCGATGACCCGTAC
CCTGAACGCCTACATGGACAAAGAAGGCTACAGCAAAAAAGCCAAAGTCAGCGCCACCGGT
GACGATGCGCGTGAAGGCCTGATTGCGGTCGTTTCCGTGAAAGTGCCGGACCCGAAATTCTC
CTCCCAGACCAAAGACAAACTGGTTTCTTCTGAGGTGAAATCGGCGGTTGAACAGCAGATGA
ACGAACTGCTGGCAGAATACCTGCTGGAAAACCCAACCGACGCGAAAATCGTGGTTGGCAA
AATTATCGATGCTGCCCGTGCCCGTGAAGCGGCGCGTCGCGCGCGTGAAATGACCCGCCGTA
AAGGTGCGCTCGACTTAGCGGGCCTGCCGGGCAAACTGGCAGACTGCCAGGAACGCGATCC
GGCGCTTTCCGAACTGTACCTGGTGGAAGGGGACTCCGCGGGCGGCTCTGCGAAGCAGGGG
CGTAACCGCAAGAACCAGGCGATTCTGCCGCTGAAGGGTAAAATCCTCAACGTCGAGAAAGC
GCGCTTCGATAAGATGCTCTCTTCTCAGGAAGTGGCGACGCTTATCACCGCGCTTGGCTGTGG
TATCGGTCGTGACGAGTACAACCCGGACAAACTGCGTTATCACAGCATCATCATCATGACCGA
TGCGGACGTCGACGGCTCGCACATTCGTACGCTGCTGTTGACCTTCTTCTATCGTCAGATGCC
GGAAATCGTTGAACGCGGTCACGTCTACATCGCTCAGCCGCCGCTGTACAAAGTGAAGAAAG
GCAAGCAGGAACAGTACATTAAAGACGACGAAGCGATGGATCAGTACCAGATCTCTATCGCG
CTGGACGGCGCAACGCTGCACACCAACGCCAGTGCACCGGCATTGGCTGGCGAAGCGTTAG
AGAAACTGGTATCTGAGTACAACGCGACGCAGAAAATGATCAATCGTATGGAGCGTCGTTATC
CGAAAGCAATGCTGAAAGAGCTTATCTATCAGCCGACGTTGACGGAAGCTGACCTTTCTGATG
AGCAGACCGTTACCCGCTGGGTGAACGCGCTGGTCAGCGAACTGAACGACAAAGAACAGCA
CGGCAGCCAGTGGAAGTTTGATGTTCACACCAATGCTGAGCAAAACCTGTTCGAGCCGATTG
TTCGCGTGCGTACCCACGGTGTGGATACTGACTATCCGCTGGATCACGAGTTTATCACCGGTG
GCGAATATCGTCGTATCTGCACGCTGGGTGAGAAACTGCGTGGCTTGCTGGAAGAAGATGCG
TTTATCGAACGTGGCGAGCGTCGTCAGCCGGTAGCCAGCTTCGAGCAGGCGCTGGACTGGCT
GGTGAAAGAGTCCCGTCGCGGCCTCTCCATCCAGCGTTATAAAGGTCTGGGCGAGATGAACC
CGGAACAGCTGTGGGAAACCACTATGGACCCGGAAAGTCGTCGTATGCTGCGCGTTACCGTT
AAAGATGCGATTGCTGCCGACCAGTTGTTCACCACGCTGATGGGCGACGCCGTTGAACCGCG
CCGTGCGTTTATTGAAGAGAACGCCCTGAAAGCGGCGAATATCGATATTTAA
SEQ ID NO. 39: Wild-type GyrB 
MSNSYDSSSIKVLKGLDAVRKRPGMYIGDTDDGTGLHHMVFEVVDNAIDEALAGHCKEIIVTIH
ADNSVSVQDDGRGIPTGIHPEEGVSAAEVIMTVLHAGGKFDDNSYKVSGGLHGVGVSVVNALS
QKLELVIQREGKIHRQIYEHGVPQAPLAVTGETEKTGTMVRFWPSLETFTNVTEFEYEILAKRLRE
LSFLNSGVSIRLRDKRDGKEDHFHYEGGIKAFVEYLNKNKTPIHPNIFYFSTEKDGIGVEVALQWN
DGFQENIYCFTNNIPQRDGGTHLAGFRAAMTRTLNAYMDKEGYSKKAKVSATGDDAREGLIAVV
SVKVPDPKFSSQTKDKLVSSEVKSAVEQQMNELLAEYLLENPTDAKIVVGKIIDAARAREAARRA
REMTRRKGALDLAGLPGKLADCQERDPALSELYLVEGDSAGGSAKQGRNRKNQAILPLKGKILN
VEKARFDKMLSSQEVATLITALGCGIGRDEYNPDKLRYHSIIIMTDADVDGSHIRTLLLTFFYRQM
PEIVERGHVYIAQPPLYKVKKGKQEQYIKDDEAMDQYQISIALDGATLHTNASAPALAGEALEKL
VSEYNATQKMINRMERRYPKAMLKELIYQPTLTEADLSDEQTVTRWVNALVSELNDKEQHGSQ
WKFDVHTNAEQNLFEPIVRVRTHGVDTDYPLDHEFITGGEYRRICTLGEKLRGLLEEDAFIERGE
RRQPVASFEQALDWLVKESRRGLSIQRYKGLGEMNPEQLWETTMDPESRRMLRVTVKDAIAADQ
LFTTLMGDAVEPRRAFIEENALKAANIDI*
SEQ ID NO. 40: GyrB Mutant 
MSNSYDSSSIKVLKGLDAVRKRPGMYIGDTDDGTGLHHMVFX1VVDNAIDEALAGHCKEIIVTIH
ADNSVSVQDDGRGIPTGIHPEEGVSAAEVIMTVLHAGGKFDDNSYKVSGGLHGVGVSVVNALS
QKLELVIQX2EGKIHRQIYEHGVPQAPLAVTGETEKTGTMVRFWPSLETFTNVTEFEYEILAKRLR
ELSFLNSGVSIRLRDKRDGKEDHFHYEGGIKAFVEYLNKNKTPIHPNIFYFSTEKDGIGVEVALQW
NDGFQENIYCFTNNIPQRDGGTHLAGFRAAMTRTLNAYMDKEGYSKKAKVSATGDDAREGLIAV
VSVKVPDPKFSSQTKDKLVSSEVKSAVEQQMNELLAEYLLENPTDAKIVVGKIIDAARAREAARR
AREMTRRKGALDLAGLPGKLADCQERDPALSELYLVEGDSAGGSAKQGRNRKNQAILPLKGKIL
NVEKARFDKMLSSQEVATLITALGCGIGRDEYNPDKLRYHSIIIMTX3ADVDGSHIRTLLLTFFYRQ
MPEIVERGHVYIAQPPLYKVKKGKQEQYIKDDEAMDQYQISIALDGATLHTNASAPALAGEALE
KLVSEYNATQKMINRMERRYPKAMLKELIYQPTLTEADLSDEQTVTRWVNALVSELNDKEQHGS
QWKFDVHTNAEQNLFEPIVRVRTHGVDTDYPLDHEFITGGEYRRICTLGEKLRGLLEEDAFIERG
ERRQPVASFEQALDWLVKESRRGLSIQRYKGLGEMNPEQLWETTMDPESRRMLRVTVKDAIAAD
QLFTTLMGDAVEPRRAFIEENALKAANIDI*
 X1 = E or D, X2 = R or C, X3 = D or A
SEQ ID NO. 3: edited *gyrA-gyrA* (H80A) coding sequence 
ATGAGCGACCTTGCGAGAGAAATTACACCGGTCAACATTGAGGAAGAGCTGAAGAGCTCCTA
TCTGGATTATGCGATGTCtGTCATTGTTGGCCGTGCGCTGCCAGATGTCCGAGATGGCCTGAAG
CCGGTACACCGTCGCGTACTTTACGCCATGAACGTACTAGGCAATGACTGGAACAAAGCCTAT
AAAAAATCTGCCCGTGTCGTTGGTGACGTAATCGGTAAATACCATCCCgcaGGTGACTCGGCGG
TCTATGACACGATCGTCCGCATGGCGCAGCCATTCTCGCTGCGTTATATGCTGGTAGACGGTCA
GGGTAACTTCGGTTCTATCGACGGCGACTCTGCGGCGGCAATGCGTTATACGGAAATCCGTCT
GGCGAAAATTGCCCATGAACTGATGGCCGATCTCGAAAAAGAGACGGTCGATTTCGTTGATA
ACTATGACGGCACGGAAAAAATTCCGGACGTCATGCCAACCAAAATTCCTAACCTGCTGGTG
AACGGTTCTTCCGGTATCGCCGTAGGTATGGCAACCAACATCCCGCCGCACAACCTGACGGA
AGTCATCAACGGTTGTCTGGCGTATATTGATGATGAAGACATCAGCATTGAAGGGCTGATGGA
ACACATCCCGGGGCCGGACTTCCCGACGGCGGCAATCATTAACGGTCGTCGCGGTATTGAAG
AAGCTTACCGTACCGGTCGCGGCAAGGTGTATATCCGCGCTCGCGCAGAAGTGGAAGTTGAC
GCCAAAACCGGTCGTGAAACCATTATCGTCCACGAAATTCCGTATCAGGTAAACAAAGCGCG
CCTGATCGAGAAGATTGCGGAACTGGTAAAAGAAAAACGCGTGGAAGGCATCAGCGCGCTG
CGTGACGAGTCTGACAAAGACGGTATGCGCATCGTGATTGAAGTGAAACGCGATGCGGTCGG
TGAAGTTGTGCTCAACAACCTCTACTCCCAGACCCAGTTGCAGGTTTCTTTCGGTATCAACAT
GGTGGCATTGCACCATGGTCAGCCGAAGATCATGAACCTGAAAGACATCATCGCGGCGTTTGT
TCGTCACCGCCGTGAAGTGGTGACCCGTCGTACTATTTTCGAACTGCGTAAAGCTCGCGATCG
 
TGCTCATATCCTTGAAGCATTAGCCGTGGCGCTGGCGAACATCGACCCGATCATCGAACTGAT
CCGTCATGCGCCGACGCCTGCAGAAGCGAAAACTGCGCTGGTTGCTAATCCGTGGCAGCTGG
GCAACGTTGCCGCGATGCTCGAACGTGCTGGCGACGATGCTGCGCGTCCGGAATGGCTGGAG
CCAGAGTTCGGCGTGCGTGATGGTCTGTACTACCTGACCGAACAGCAAGCTCAGGCGATTCT
GGATCTGCGTTTGCAGAAACTGACCGGTCTTGAGCACGAAAAACTGCTCGACGAATACAAAG
AGCTGCTGGATCAGATCGCGGAACTGTTGCGTATTCTTGGTAGCGCCGATCGTCTGATGGAAG
TGATCCGTGAAGAGCTGGAGCTGGTTCGTGAACAGTTCGGTGACAAACGTCGTACTGAAATC
ACCGCCAACAGCGCAGACATCAACCTGGAAGATCTGATCACCCAGGAAGATGTGGTCGTGAC
GCTCTCTCACCAGGGCTACGTTAAGTATCAGCCGCTTTCTGAATACGAAGCGCAGCGTCGTGG
CGGGAAAGGTAAATCTGCCGCACGTATTAAAGAAGACTTTATCGACCGACTGCTGGTGG
CGAACACTCACGACCATATTCTGTGCTTCTCCAGCCGTGGTCGCGTCTATTCGATGAAAGTTTA
TCAGTTGCCGGAAGCCACTCGTGGCGCGCGCGGTCGTCCGATCGTCAACCTGCTGCCGCTGG
AGCAGGACGAACGTATCACTGCGATCCTGCCAGTGACCGAGTTTGAAGAAGGCGTGAAAGTC
TTCATGGCGACCGCTAACGGTACCGTGAAGAAAACTGTCCTCACCGAGTTCAACCGTCTGCG
TACCGCCGGTAAAGTGGCGATCAAACTGGTTGACGGCGATGAGCTGATCGGCGTTGACCTGA
CCAGCGGCGAAGACGAAGTAATGCTGTTCTCCGCTGAAGGTAAAGTGGTGCGCTTTAAAGAG
TCTTCTGTCCGTGCGATGGGCTGCAACACCACCGGTGTTCGCGGTATTCGCTTAGGTGAAGGC
GATAAAGTCGTCTCTCTGATCGTGCCTCGTGGCGATGGCGCAATCCTCACCGCAACGCAAAAC
GGTTACGGTAAACGTACCGCAGTGGCGGAATACCCAACCAAGTCGCGTGCGACGAAAGGGGT
TATCTCCATCAAGGTTACCGAACGTAACGGTTTAGTTGTTGGCGCGGTACAGGTAGATGACTG
CGACCAGATCATGATGATCACCGATGCCGGTACGCTGGTACGTACTCGCGTTTCGGAAATCAG
CATCGTGGGCCGTAACACCCAGGGCGTGATCCTCATCCGTACTGCGGAAGATGAAAACGTAG
TGGGTCTGCAACGTGTTGCTGAACCGGTTGACGAGGAAGATCTGGATACCATCGACGGCAGT
GCCGCGGAAGGGGACGATGAAATCGCTCCGGAAGTGGACGTTGACGACGAGCCAGAAGAAG
AATAA
SEQ ID NO. 4: edited *gyrA-gyrA* (A569T&T586A) coding sequence 
ATGAGCGACCTTGCGAGAGAAATTACACCGGTCAACATTGAGGAAGAGCTGAAGAGCTCCTA
TCTGGATTATGCGATGTCGGTCATTGTTGGCCGTGCGCTGCCAGATGTCCGAGATGGCCTGAA
GCCGGTACACCGTCGCGTACTTTACGCCATGAACGTACTAGGCAATGACTGGAACAAAGCCTA
TAAAAAATCTGCCCGTGTCGTTGGTGACGTAATCGGTAAATACCATCCCCATGGTGACTCGGC
GGTCTATGACACGATCGTCCGCATGGCGCAGCCATTCTCGCTGCGTTATATGCTGGTAGACGGT
CAGGGTAACTTCGGTTCTATCGACGGCGACTCTGCGGCGGCAATGCGTTATACGGAAATCCGT
CTGGCGAAAATTGCCCATGAACTGATGGCCGATCTCGAAAAAGAGACGGTCGATTTCGTTGAT
AACTATGACGGCACGGAAAAAATTCCGGACGTCATGCCAACCAAAATTCCTAACCTGCTGGT
GAACGGTTCTTCCGGTATCGCCGTAGGTATGGCAACCAACATCCCGCCGCACAACCTGACGG
AAGTCATCAACGGTTGTCTGGCGTATATTGATGATGAAGACATCAGCATTGAAGGGCTGATGG
AACACATCCCGGGGCCGGACTTCCCGACGGCGGCAATCATTAACGGTCGTCGCGGTATTGAA
GAAGCTTACCGTACCGGTCGCGGCAAGGTGTATATCCGCGCTCGCGCAGAAGTGGAAGTTGA
CGCCAAAACCGGTCGTGAAACCATTATCGTCCACGAAATTCCGTATCAGGTAAACAAAGCGC
GCCTGATCGAGAAGATTGCGGAACTGGTAAAAGAAAAACGCGTGGAAGGCATCAGCGCGCT
GCGTGACGAGTCTGACAAAGACGGTATGCGCATCGTGATTGAAGTGAAACGCGATGCGGTCG
GTGAAGTTGTGCTCAACAACCTCTACTCCCAGACCCAGTTGCAGGTTTCTTTCGGTATCAACA
TGGTGGCATTGCACCATGGTCAGCCGAAGATCATGAACCTGAAAGACATCATCGCGGCGTTTG
TTCGTCACCGCCGTGAAGTGGTGACCCGTCGTACTATTTTCGAACTGCGTAAAGCTCGCGATC
GTGCTCATATCCTTGAAGCATTAGCCGTGGCGCTGGCGAACATCGACCCGATCATCGAACTGA
TCCGTCATGCGCCGACGCCTGCAGAAGCGAAAACTGCGCTGGTTGCTAATCCGTGGCAGCTG
GGCAACGTTGCCGCGATGCTCGAACGTGCTGGCGACGATGCTGCGCGTCCGGAATGGCTGGA
GCCAGAGTTCGGCGTGCGTGATGGTCTGTACTACCTGACCGAACAGCAAGCTCAGGCGATTC
 
TGGATCTGCGTTTGCAGAAACTGACCGGTCTTGAGCACGAAAAACTGCTCGACGAATACAAA
GAGCTGCTGGATCAGATCGCGGAACTGTTGCGTATTCTTGGTAGCGCCGATCGTCTGATGGAA
GTGATCCGTGAAGAGCTGGAGCTGGTTCGTGAACAGTTCGGTGACAAACGTCGTACTGAAAT
CACCGCCAACAGCGCAGACATCAACCTcGAAGATCTGATCACCCAGGAAGATGTGGTCGTGA
CGCTCTCTCACCAGGGCTACGTTAAGTATCAGCCGCTTTCTGAATACGAAGCGCAGCGTCGTG
GCGGGAAAGGTAAATCTactGCACGTATTAAAGAAGACTTTATCGACCGACTGCTGGTGGC
GAACgccCACGACCATATTCTGTGCTTCTCCAGCCGTGGTCGCGTCTATTCGATGAAAGTTTATC
AGTTGCCGGAAGCCACTCGTGGCGCGCGCGGTCGTCCGATCGTCAACCTGCTGCCGCTGGAG
CAGGACGAACGTATCACTGCGATCCTGCCAGTGACCGAGTTTGAAGAAGGCGTGAAAGTCTT
CATGGCGACCGCTAACGGTACCGTGAAGAAAACTGTCCTCACCGAGTTCAACCGTCTGCGTA
CCGCCGGTAAAGTGGCGATCAAACTGGTTGACGGCGATGAGCTGATCGGCGTTGACCTGACC
AGCGGCGAAGACGAAGTAATGCTGTTCTCCGCTGAAGGTAAAGTGGTGCGCTTTAAAGAGTC
TTCTGTCCGTGCGATGGGCTGCAACACCACCGGTGTTCGCGGTATTCGCTTAGGTGAAGGCGA
TAAAGTCGTCTCTCTGATCGTGCCTCGTGGCGATGGCGCAATCCTCACCGCAACGCAAAACG
GTTACGGTAAACGTACCGCAGTGGCGGAATACCCAACCAAGTCGCGTGCGACGAAAGGGGTT
ATCTCCATCAAGGTTACCGAACGTAACGGTTTAGTTGTTGGCGCGGTACAGGTAGATGACTGC
GACCAGATCATGATGATCACCGATGCCGGTACGCTGGTACGTACTCGCGTTTCGGAAATCAGC
ATCGTGGGCCGTAACACCCAGGGCGTGATCCTCATCCGTACTGCGGAAGATGAAAACGTAGT
GGGTCTGCAACGTGTTGCTGAACCGGTTGACGAGGAAGATCTGGATACCATCGACGGCAGTG
CCGCGGAAGGGGACGATGAAATCGCTCCGGAAGTGGACGTTGACGACGAGCCAGAAGAAGA
ATAA
SEQ ID NO. 5: edited *gyrB-gyrB* (E42D) coding sequence 
ATGTCGAATTCTTATGACTCCTCCAGTATCAAAGTCCTGAAAGGGCTGGATGCGGTGCGTAAG
CGCCCGGGTATGTATATCGGCGACACGGATGACGGCACCGGTCTGCACCACATGGTATTCGAtG
TGGTAGATAACGCTATCGACGAAGCGCTCGCcGGTCACTGTAAAGAAATTATCGTCACCATTCA
CGCCGATAACTCTGTCTCTGTACAGGATGACGGGCGCGGCATTCCGACCGGTATTCACCCGGA
AGAGGGCGTATCGGCGGCGGAAGTGATCATGACCGTTCTGCACGCAGGCGGTAAATTTGACG
ATAACTCCTATAAAGTGTCCGGCGGTCTGCACGGCGTTGGTGTTTCGGTAGTAAACGCCCTGT
CGCAAAAACTGGAGCTGGTTATCCAGCGCGAGGGTAAAATTCACCGTCAGATCTACGAACAC
GGTGTACCGCAGGCCCCGCTGGCGGTTACCGGCGAGACTGAAAAAACCGGCACCATGGTGC
GTTTCTGGCCCAGCCTCGAAACCTTCACCAATGTGACCGAGTTCGAATATGAAATTCTGGCGA
AACGTCTGCGTGAGTTGTCGTTCCTCAACTCCGGCGTTTCCATTCGTCTGCGCGACAAGCGCG
ACGGCAAAGAAGACCACTTCCACTATGAAGGCGGCATCAAGGCGTTCGTTGAATATCTGAAC
AAGAACAAAACGCCGATCCACCCGAATATCTTCTACTTCTCCACTGAAAAAGACGGTATTGGC
GTCGAAGTGGCGTTGCAGTGGAACGATGGCTTCCAGGAAAACATCTACTGCTTTACCAACAA
CATTCCGCAGCGTGACGGCGGTACTCACCTGGCAGGCTTCCGTGCGGCGATGACCCGTACCCT
GAACGCCTACATGGACAAAGAAGGCTACAGCAAAAAAGCCAAAGTCAGCGCCACCGGTGAC
GATGCGCGTGAAGGCCTGATTGCGGTCGTTTCCGTGAAAGTGCCGGACCCGAAATTCTCCTCC
CAGACCAAAGACAAACTGGTTTCTTCTGAGGTGAAATCGGCGGTTGAACAGCAGATGAACG
AACTGCTGGCAGAATACCTGCTGGAAAACCCAACCGACGCGAAAATCGTGGTTGGCAAAATT
ATCGATGCTGCCCGTGCCCGTGAAGCGGCGCGTCGCGCGCGTGAAATGACCCGCCGTAAAGG
TGCGCTCGACTTAGCGGGCCTGCCGGGCAAACTGGCAGACTGCCAGGAACGCGATCCGGCG
CTTTCCGAACTGTACCTGGTGGAAGGGGACTCCGCGGGCGGCTCTGCGAAGCAGGGGCGTAA
CCGCAAGAACCAGGCGATTCTGCCGCTGAAGGGTAAAATCCTCAACGTCGAGAAAGCGCGCT
TCGATAAGATGCTCTCTTCTCAGGAAGTGGCGACGCTTATCACCGCGCTTGGCTGTGGTATCG
GTCGTGACGAGTACAACCCGGACAAACTGCGTTATCACAGCATCATCATCATGACCGATGCGG
ACGTCGACGGCTCGCACATTCGTACGCTGCTGTTGACCTTCTTCTATCGTCAGATGCCGGAAA
TCGTTGAACGCGGTCACGTCTACATCGCTCAGCCGCCGCTGTACAAAGTGAAGAAAGGCAAG
 
CAGGAACAGTACATTAAAGACGACGAAGCGATGGATCAGTACCAGATCTCTATCGCGCTGGA
CGGCGCAACGCTGCACACCAACGCCAGTGCACCGGCATTGGCTGGCGAAGCGTTAGAGAAA
CTGGTATCTGAGTACAACGCGACGCAGAAAATGATCAATCGTATGGAGCGTCGTTATCCGAAA
GCAATGCTGAAAGAGCTTATCTATCAGCCGACGTTGACGGAAGCTGACCTTTCTGATGAGCAG
ACCGTTACCCGCTGGGTGAACGCGCTGGTCAGCGAACTGAACGACAAAGAACAGCACGGCA
GCCAGTGGAAGTTTGATGTTCACACCAATGCTGAGCAAAACCTGTTCGAGCCGATTGTTCGC
GTGCGTACCCACGGTGTGGATACTGACTATCCGCTGGATCACGAGTTTATCACCGGTGGCGAA
TATCGTCGTATCTGCACGCTGGGTGAGAAACTGCGTGGCTTGCTGGAAGAAGATGCGTTTATC
GAACGTGGCGAGCGTCGTCAGCCGGTAGCCAGCTTCGAGCAGGCGCTGGACTGGCTGGTGA
AAGAGTCCCGTCGCGGCCTCTCCATCCAGCGTTATAAAGGTCTGGGCGAGATGAACCCGGAA
CAGCTGTGGGAAACCACTATGGACCCGGAAAGTCGTCGTATGCTGCGCGTTACCGTTAAAGAT
GCGATTGCTGCCGACCAGTTGTTCACCACGCTGATGGGCGACGCCGTTGAACCGCGCCGTGC
GTTTATTGAAGAGAACGCCCTGAAAGCGGCGAATATCGATATTTAA
SEQ ID NO. 6: edited *gyrB-gyrB* (R136C) coding sequence 
ATGTCGAATTCTTATGACTCCTCCAGTATCAAAGTCCTGAAAGGGCTGGATGCGGTGCGTAAG
CGCCCGGGTATGTATATCGGCGACACGGATGACGGCACCGGTCTGCACCACATGGTATTCGAG
GTGGTAGATAACGCTATCGACGAAGCGCTCGCGGGTCACTGTAAAGAAATTATCGTCACCATT
CACGCCGATAACTCTGTCTCTGTACAGGATGACGGGCGCGGCATTCCGACCGGTATTCACCCG
GAAGAGGGCGTATCGGCcGCGGAAGTGATCATGACCGTTCTGCACGCAGGCGGTAAATTTGA
CGATAACTCCTATAAAGTGTCCGGCGGTCTGCACGGCGTTGGTGTTTCGGTAGTAAACGCCCT
GTCGCAAAAACTGGAGCTGGTTATCCAGtGCGAGGGTAAAATTCACCGTCAGATCTACGAACA
CGGTGTACCGCAGGCCCCGCTGGCGGTTACCGGCGAGACTGAAAAAACCGGCACCATGGTGC
GTTTCTGGCCCAGCCTCGAAACCTTCACCAATGTGACCGAGTTCGAATATGAAATTCTGGCGA
AACGTCTGCGTGAGTTGTCGTTCCTCAACTCCGGCGTTTCCATTCGTCTGCGCGACAAGCGCG
ACGGCAAAGAAGACCACTTCCACTATGAAGGCGGCATCAAGGCGTTCGTTGAATATCTGAAC
AAGAACAAAACGCCGATCCACCCGAATATCTTCTACTTCTCCACTGAAAAAGACGGTATTGGC
GTCGAAGTGGCGTTGCAGTGGAACGATGGCTTCCAGGAAAACATCTACTGCTTTACCAACAA
CATTCCGCAGCGTGACGGCGGTACTCACCTGGCAGGCTTCCGTGCGGCGATGACCCGTACCCT
GAACGCCTACATGGACAAAGAAGGCTACAGCAAAAAAGCCAAAGTCAGCGCCACCGGTGAC
GATGCGCGTGAAGGCCTGATTGCGGTCGTTTCCGTGAAAGTGCCGGACCCGAAATTCTCCTCC
CAGACCAAAGACAAACTGGTTTCTTCTGAGGTGAAATCGGCGGTTGAACAGCAGATGAACG
AACTGCTGGCAGAATACCTGCTGGAAAACCCAACCGACGCGAAAATCGTGGTTGGCAAAATT
ATCGATGCTGCCCGTGCCCGTGAAGCGGCGCGTCGCGCGCGTGAAATGACCCGCCGTAAAGG
TGCGCTCGACTTAGCGGGCCTGCCGGGCAAACTGGCAGACTGCCAGGAACGCGATCCGGCG
CTTTCCGAACTGTACCTGGTGGAAGGGGACTCCGCGGGCGGCTCTGCGAAGCAGGGGCGTAA
CCGCAAGAACCAGGCGATTCTGCCGCTGAAGGGTAAAATCCTCAACGTCGAGAAAGCGCGCT
TCGATAAGATGCTCTCTTCTCAGGAAGTGGCGACGCTTATCACCGCGCTTGGCTGTGGTATCG
GTCGTGACGAGTACAACCCGGACAAACTGCGTTATCACAGCATCATCATCATGACCGATGCGG
ACGTCGACGGCTCGCACATTCGTACGCTGCTGTTGACCTTCTTCTATCGTCAGATGCCGGAAA
TCGTTGAACGCGGTCACGTCTACATCGCTCAGCCGCCGCTGTACAAAGTGAAGAAAGGCAAG
CAGGAACAGTACATTAAAGACGACGAAGCGATGGATCAGTACCAGATCTCTATCGCGCTGGA
CGGCGCAACGCTGCACACCAACGCCAGTGCACCGGCATTGGCTGGCGAAGCGTTAGAGAAA
CTGGTATCTGAGTACAACGCGACGCAGAAAATGATCAATCGTATGGAGCGTCGTTATCCGAAA
GCAATGCTGAAAGAGCTTATCTATCAGCCGACGTTGACGGAAGCTGACCTTTCTGATGAGCAG
ACCGTTACCCGCTGGGTGAACGCGCTGGTCAGCGAACTGAACGACAAAGAACAGCACGGCA
GCCAGTGGAAGTTTGATGTTCACACCAATGCTGAGCAAAACCTGTTCGAGCCGATTGTTCGC
GTGCGTACCCACGGTGTGGATACTGACTATCCGCTGGATCACGAGTTTATCACCGGTGGCGAA
TATCGTCGTATCTGCACGCTGGGTGAGAAACTGCGTGGCTTGCTGGAAGAAGATGCGTTTATC
 
GAACGTGGCGAGCGTCGTCAGCCGGTAGCCAGCTTCGAGCAGGCGCTGGACTGGCTGGTGA
AAGAGTCCCGTCGCGGCCTCTCCATCCAGCGTTATAAAGGTCTGGGCGAGATGAACCCGGAA
CAGCTGTGGGAAACCACTATGGACCCGGAAAGTCGTCGTATGCTGCGCGTTACCGTTAAAGAT
GCGATTGCTGCCGACCAGTTGTTCACCACGCTGATGGGCGACGCCGTTGAACCGCGCCGTGC
GTTTATTGAAGAGAACGCCCTGAAAGCGGCGAATATCGATATTTAA
SEQ ID NO. 7: edited *gyrB-gyrB* (D498A) coding sequence 
ATGTCGAATTCTTATGACTCCTCCAGTATCAAAGTCCTGAAAGGGCTGGATGCGGTGCGTAAG
CGCCCGGGTATGTATATCGGCGACACGGATGACGGCACCGGTCTGCACCACATGGTATTCGAG
GTGGTAGATAACGCTATCGACGAAGCGCTCGCGGGTCACTGTAAAGAAATTATCGTCACCATT
CACGCCGATAACTCTGTCTCTGTACAGGATGACGGGCGCGGCATTCCGACCGGTATTCACCCG
GAAGAGGGCGTATCGGCGGCGGAAGTGATCATGACCGTTCTGCACGCAGGCGGTAAATTTGA
CGATAACTCCTATAAAGTGTCCGGCGGTCTGCACGGCGTTGGTGTTTCGGTAGTAAACGCCCT
GTCGCAAAAACTGGAGCTGGTTATCCAGCGCGAGGGTAAAATTCACCGTCAGATCTACGAAC
ACGGTGTACCGCAGGCCCCGCTGGCGGTTACCGGCGAGACTGAAAAAACCGGCACCATGGT
GCGTTTCTGGCCCAGCCTCGAAACCTTCACCAATGTGACCGAGTTCGAATATGAAATTCTGGC
GAAACGTCTGCGTGAGTTGTCGTTCCTCAACTCCGGCGTTTCCATTCGTCTGCGCGACAAGCG
CGACGGCAAAGAAGACCACTTCCACTATGAAGGCGGCATCAAGGCGTTCGTTGAATATCTGA
ACAAGAACAAAACGCCGATCCACCCGAATATCTTCTACTTCTCCACTGAAAAAGACGGTATTG
GCGTCGAAGTGGCGTTGCAGTGGAACGATGGCTTCCAGGAAAACATCTACTGCTTTACCAAC
AACATTCCGCAGCGTGACGGCGGTACTCACCTGGCAGGCTTCCGTGCGGCGATGACCCGTAC
CCTGAACGCCTACATGGACAAAGAAGGCTACAGCAAAAAAGCCAAAGTCAGCGCCACCGGT
GACGATGCGCGTGAAGGCCTGATTGCGGTCGTTTCCGTGAAAGTGCCGGACCCGAAATTCTC
CTCCCAGACCAAAGACAAACTGGTTTCTTCTGAGGTGAAATCGGCGGTTGAACAGCAGATGA
ACGAACTGCTGGCAGAATACCTGCTGGAAAACCCAACCGACGCGAAAATCGTGGTTGGCAA
AATTATCGATGCTGCCCGTGCCCGTGAAGCGGCGCGTCGCGCGCGTGAAATGACCCGCCGTA
AAGGTGCGCTCGACTTAGCGGGCCTGCCGGGCAAACTGGCAGACTGCCAGGAACGCGATCC
GGCGCTTTCCGAACTGTACCTGGTGGAAGGGGACTCCGCGGGCGGCTCTGCGAAGCAGGGG
CGTAACCGCAAGAACCAGGCGATTCTGCCGCTGAAGGGTAAAATCCTCAACGTCGAGAAAGC
GCGCTTCGATAAGATGCTCTCTTCTCAaGAAGTGGCGACGCTTATCACCGCGCTTGGCTGTGGT
ATCGGTCGTGACGAGTACAACCCGGACAAACTGCGTTATCACAGCATCATCATCATGACCGccG
CGGACGTCGACGGCTCGCACATTCGTACGCTGCTGTTGACCTTCTTCTATCGTCAGATGCCGG
AAATCGTTGAACGCGGTCACGTCTACATCGCTCAGCCGCCGCTGTACAAAGTGAAGAAAGGC
AAGCAGGAACAGTACATTAAAGACGACGAAGCGATGGATCAGTACCAGATCTCTATCGCGCT
GGACGGCGCAACGCTGCACACCAACGCCAGTGCACCGGCATTGGCTGGCGAAGCGTTAGAG
AAACTGGTATCTGAGTACAACGCGACGCAGAAAATGATCAATCGTATGGAGCGTCGTTATCCG
AAAGCAATGCTGAAAGAGCTTATCTATCAGCCGACGTTGACGGAAGCTGACCTTTCTGATGAG
CAGACCGTTACCCGCTGGGTGAACGCGCTGGTCAGCGAACTGAACGACAAAGAACAGCACG
GCAGCCAGTGGAAGTTTGATGTTCACACCAATGCTGAGCAAAACCTGTTCGAGCCGATTGTT
CGCGTGCGTACCCACGGTGTGGATACTGACTATCCGCTGGATCACGAGTTTATCACCGGTGGC
GAATATCGTCGTATCTGCACGCTGGGTGAGAAACTGCGTGGCTTGCTGGAAGAAGATGCGTTT
ATCGAACGTGGCGAGCGTCGTCAGCCGGTAGCCAGCTTCGAGCAGGCGCTGGACTGGCTGGT
GAAAGAGTCCCGTCGCGGCCTCTCCATCCAGCGTTATAAAGGTCTGGGCGAGATGAACCCGG
AACAGCTGTGGGAAACCACTATGGACCCGGAAAGTCGTCGTATGCTGCGCGTTACCGTTAAA
GATGCGATTGCTGCCGACCAGTTGTTCACCACGCTGATGGGCGACGCCGTTGAACCGCGCCG
TGCGTTTATTGAAGAGAACGCCCTGAAAGCGGCGAATATCGATATTTAA
SEQ ID NO. 8: sgRNA targeting *gyrA* (H80A)
   ctatctggattatgcgatgt
SEQ ID NO. 9: sgRNA targeting *gyrA* gene (A569T&T586A)
   caacagcgcagacatcaacc
SEQ ID NO. 10: sgRNA targeting *gyrB* gene (E42D)
   cgctatcgacgaagcgctcg
SEQ ID NO. 11: sgRNA targeting *gyrB* gene (R136C)
   cccggaagagggcgtatcgg
SEQ ID NO. 12: sgRNA targeting *gyrB* gene (D498A)
   cgataagatgctctcttctc
SEQ ID NO. 13: *gyrA* (H80A) donor sequence 
GCGGTTTGAACTCACCTTCCAGATCCCACCAGCGGGAGGCGACGGCTTCAAATTTAGCGATCT
CTTCGTGGTCTACGTTATGGTTTACCGGCGATTTTTCGGCATTCATTGGCACTTCTACTCCGTAA
TTGGCAAGACAAACGAGTATATCAGGCATTGGATGTGAATAAAGCGTATAGGTTTACCTCAAA
CTGCGCGGCTGTGTTATAATTTGCGACCTTTGAATCCGGGATACAGTAGAGGGATAGCGGTTA
GATGAGCGACCTTGCGAGAGAAATTACACCGGTCAACATTGAGGAAGAGCTGAAGAGCTCCT
ATCTGGATTATGCGATGTCtGTCATTGTTGGCCGTGCGCTGCCAGATGTCCGAGATGGCCTGAA
GCCGGTACACCGTCGCGTACTTTACGCCATGAACGTACTAGGCAATGACTGGAACAAAGCCTA
TAAAAAATCTGCCCGTGTCGTTGGTGACGTAATCGGTAAATACCATCCCgcaGGTGACTCGGCG
GTCTATGACACGATCGTCCGCATGGCGCAGCCATTCTCGCTGCGTTATATGCTGGTAGACGGTC
AGGGTAACTTCGGTTCTATCGACGGCGACTCTGCGGCGGCAATGCGTTATACGGAAATCCGTC
TGGCGAAAATTGCCCATGAACTGATGGCCGATCTCGAAAAAGAGACGGTCGATTTCGTTGATA
ACTATGACGGCACGGAAAAAATTCCGGACGTCATGCCAACCAAAATTCCTAACCTGCTGGTG
AACGGTTCTTCCGGTATCGCCGTAGGTATGGCAACCAACATCCCGCCGCACAACCTGACGGA
AGTCATCAACGGTTGTCTGGCGTATATTGATGATGAAGACATCAGCATTGAAGGGCTGATGGA
ACACATCCCGGGGCCGGACTTCCCGACGGCGGCAATCATTAACGGTCGTCGCGGTATTGAAG
AAGCTTACCGTACCGGTCGCGGCAAGGTGTATATCCGCGCTCGCGCAGAAGTGG
SEQ ID NO. 14: *gyrA* (A569T&T586A) donor sequence 
GTTCGGCGTGCGTGATGGTCTGTACTACCTGACCGAACAGCAAGCTCAGGCGATTCTGGATCT
GCGTTTGCAGAAACTGACCGGTCTTGAGCACGAAAAACTGCTCGACGAATACAAAGAGCTG
CTGGATCAGATCGCGGAACTGTTGCGTATTCTTGGTAGCGCCGATCGTCTGATGGAAGTGATC
CGTGAAGAGCTGGAGCTGGTTCGTGAACAGTTCGGTGACAAACGTCGTACTGAAATCACCGC
CAACAGCGCAGACATCAACCTcGAAGATCTGATCACCCAGGAAGATGTGGTCGTGACGCTCTC
TCACCAGGGCTACGTTAAGTATCAGCCGCTTTCTGAATACGAAGCGCAGCGTCGTGGCGGGA
AAGGTAAATCTactGCACGTATTAAAGAAGAAGACTTTATCGACCGACTGCTGGTGGCGAACgcc
CACGACCATATTCTGTGCTTCTCCAGCCGTGGTCGCGTCTATTCGATGAAAGTTTATCAGTTGC
CGGAAGCCACTCGTGGCGCGCGCGGTCGTCCGATCGTCAACCTGCTGCCGCTGGAGCAGGAC
GAACGTATCACTGCGATCCTGCCAGTGACCGAGTTTGAAGAAGGCGTGAAAGTCTTCATGGC
GACCGCTAACGGTACCGTGAAGAAAACTGTCCTCACCGAGTTCAACCGTCTGCGTACCGCCG
GTAAAGTGGCGATCAAACTGGTTGACGGCGATGAGCTGATCGGCGTTGACCTGACCAGCGGC
GAAGACGAAGTAATGCTGTTCTCCGCTGAAGGTAAAGTGGTGCGCTTTAAAGAGTCTTCTGT
CCGTGCGATGGGCTGCAACACCACCGGTGTTCGCGGTATTCGCTTAGGTGAAGGCGATAAAG
TCGTCTCTCTGATCGTGCCTCGTGGCGATGGCGCAATCCTCACCGCAACGCAAAACGGTTACG
GTAAACGTACCGCAGTGGCGGAATACCCAACCAAGTCGCGTGCGACGAAAGGGGTTATCTCC
SEQ ID NO. 15: *gyrB* (E42D) donor sequence 
CGACGGTGCGCCGGTGGAAGATACCTTATCGCGTGGGCAGCTTAAGCTGTTGATGTGCGCCTT
ACGTCTGGCGCAAGGAGAGTTCCTCACCCGTGAAAGCGGGCGGCGGTGTCTCTACCTGATAG
ATGATTTTGCCTCTGAGCTTGATGATGAGCGTCGCGGGCTGCTTGCCAGCCGCTTAAAAGCGA
CGCAATCACAGGTCTTTGTCAGCGCGATCAGTGCTGAACACGTTATAGACATGTCGGACGAAA
ATTCGAAGATGTTTACCGTGGAAAAGGGTAAAATAACGGATTAACCCAAGTATAAATGAGCGA
GAAACGTTGATGTCGAATTCTTATGACTCCTCCAGTATCAAAGTCCTGAAAGGGCTGGATGCG
GTGCGTAAGCGCCCGGGTATGTATATCGGCGACACGGATGACGGCACCGGTCTGCACCACATG
GTATTCGAtGTGGTAGATAACGCTATCGACGAAGCGCTCGCcGGTCACTGTAAAGAAATTATCG
TCACCATTCACGCCGATAACTCTGTCTCTGTACAGGATGACGGGCGCGGCATTCCGACCGGTA
TTCACCCGGAAGAGGGCGTATCGGCGGCGGAAGTGATCATGACCGTTCTGCACGCAGGCGGT
AAATTTGACGATAACTCCTATAAAGTGTCCGGCGGTCTGCACGGCGTTGGTGTTTCGGTAGTA
AACGCCCTGTCGCAAAAACTGGAGCTGGTTATCCAGCGCGAGGGTAAAATTCACCGTCAGAT
CTACGAACACGGTGTACCGCAGGCCCCGCTGGCGGTTACCGGCGAGACTGAAAAAACCGGC
ACCATGGTGCGTTTCTGGCCCAGCCTCGAAACCTTCACCAATGTGACCGAGTTCGAATATGAA
ATTCTGGCGAAACGTCTGCGTGAGTTGTCGTTCCTCAACTCCGGCGTTTCCATTCGTCTGCGC
GACAAGCGCGACGGCAAAGAAGACCACTTCCACTATGAAGGCGGCATCAAGGCGTTCGT
SEQ ID NO. 16: *gyrB* (R136C) donor sequence 
CGTTGATGTCGAATTCTTATGACTCCTCCAGTATCAAAGTCCTGAAAGGGCTGGATGCGGTGC
GTAAGCGCCCGGGTATGTATATCGGCGACACGGATGACGGCACCGGTCTGCACCACATGGTAT
TCGAGGTGGTAGATAACGCTATCGACGAAGCGCTCGCGGGTCACTGTAAAGAAATTATCGTCA
CCATTCACGCCGATAACTCTGTCTCTGTACAGGATGACGGGCGCGGCATTCCGACCGGTATTC
ACCCGGAAGAGGGCGTATCGGCcGCGGAAGTGATCATGACCGTTCTGCACGCAGGCGGTAAA
TTTGACGATAACTCCTATAAAGTGTCCGGCGGTCTGCACGGCGTTGGTGTTTCGGTAGTAAAC
GCCCTGTCGCAAAAACTGGAGCTGGTTATCCAGtGCGAGGGTAAAATTCACCGTCAGATCTAC
GAACACGGTGTACCGCAGGCCCCGCTGGCGGTTACCGGCGAGACTGAAAAAACCGGCACCA
TGGTGCGTTTCTGGCCCAGCCTCGAAACCTTCACCAATGTGACCGAGTTCGAATATGAAATTC
TGGCGAAACGTCTGCGTGAGTTGTCGTTCCTCAACTCCGGCGTTTCCATTCGTCTGCGCGACA
AGCGCGACGGCAAAGAAGACCACTTCCACTATGAAGGCGGCATCAAGGCGTTCGTTGAATAT
CTGAACAAGAACAAAACGCCGATCCACCCGAATATCTTCTACTTCTCCACTGAAAAAGACGG
TATTGGCGTCGAAGTGGCGTTGCAGTGGAACGATGGCTTCCAGGAAAACATCTACTGCTTTAC
CAACAACATTCCGCAGCGTGACGGCGGTACTCACCTGGCAGGCTTCCGTGCGGCGATGACCC
GTACCCTGAACGCCTACATGGACAAAGAAGGCTACAGCAAAAAAGCCAAAGTCAGCGCCAC
CGGTGACGATGCGCGTGAAGGCCTGATTGCGGTCGTTTCCGTGAAAGTGCCGGACCCGAAAT
T
SEQ ID NO. 17: *gyrB* (D498A) donor sequence 
TGAACGAACTGCTGGCAGAATACCTGCTGGAAAACCCAACCGACGCGAAAATCGTGGTTGGC
AAAATTATCGATGCTGCCCGTGCCCGTGAAGCGGCGCGTCGCGCGCGTGAAATGACCCGCCG
TAAAGGTGCGCTCGACTTAGCGGGCCTGCCGGGCAAACTGGCAGACTGCCAGGAACGCGATC
CGGCGCTTTCCGAACTGTACCTGGTGGAAGGGGACTCCGCGGGCGGCTCTGCGAAGCAGGG
GCGTAACCGCAAGAACCAGGCGATTCTGCCGCTGAAGGGTAAAATCCTCAACGTCGAGAAAG
CGCGCTTCGATAAGATGCTCTCTTCTCAaGAAGTGGCGACGCTTATCACCGCGCTTGGCTGTG
GTATCGGTCGTGACGAGTACAACCCGGACAAACTGCGTTATCACAGCATCATCATCATGACCG
ccGCGGACGTCGACGGCTCGCACATTCGTACGCTGCTGTTGACCTTCTTCTATCGTCAGATGCC
GGAAATCGTTGAACGCGGTCACGTCTACATCGCTCAGCCGCCGCTGTACAAAGTGAAGAAAG
GCAAGCAGGAACAGTACATTAAAGACGACGAAGCGATGGATCAGTACCAGATCTCTATCGCG
 
CTGGACGGCGCAACGCTGCACACCAACGCCAGTGCACCGGCATTGGCTGGCGAAGCGTTAG
AGAAACTGGTATCTGAGTACAACGCGACGCAGAAAATGATCAATCGTATGGAGCGTCGTTATC
CGAAAGCAATGCTGAAAGAGCTTATCTATCAGCCGACGTTGACGGAAGCTGACCTTTCTGATG
AGCAGACCGTTACCCGCTGGGTGAACGCGCTGGTCAGCGAACTGAACGACAAAGAACAGCA
CGGCAGCCAGTGGAAGTTTGATGTTCACACCAATGCTGAGCAAAACCTGTTCGAGCCGATTG
TTCGCGTGCGTACCCACGGTGTGGATACTGACTATCCGCTGGATCACGAGTTTATCACCGGTG
GCGA
SEQ ID NO. 30: poly(A) sequence-80A
   aaaaaaaaaaaaaaaaaaaaaaaaaaaaaaaaaaaaaaaaaaaaaaaaaaaaaaaaaaaaaaaaaaaaaaaaaaaaaaaa
SEQ ID NO. 31: poly(A) sequence-100A 
aaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaa
aaaaa
SEQ ID NO. 32: poly(A) sequence-120A 
aaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaa
aaaaaaaaaaaaaaaaaaaaaaaaaa
SEQ ID NO. 33: poly(A) sequence-30&70A 
aaaaaaaaaaaaaaaaaaaaaaaaaaaagcatatgactaaaaaaaaaaaaaaaaaaaaaaaaaaaaaaaaaaaaaaaaaaaaaaaaaaaaaaaaaaa
aaaaaaaaaaaaaa
SEQ ID NO. 34: poly(A) sequence-30&30&43A 
aaaaaaaaaaaaaaaaaaaaaaaaaaaaaagcatatgactaaaaaaaaaaaaaaaaaaaaaaaaaaaaaagcatatgactaaaaaaaaaaaaaaaaa
aaaaaaaaaaaaaaaaaaaaaaaaaa
SEQ ID NO. 35: mRNA functional region sequence of poly(A)-Test 1 (80A) 
taatacgactcactataaggggtcttctggtccccacagactcagagagaacccgccaccatggtgagcaagggcgaggagctgttcaccggggtggt
gcccatcctggtcgagctggacggcgacgtaaacggccacaagttcagcgtgtccggcgagggcgagggcgatgccacctacggcaagctgaccct
gaagttcatctgcaccaccggcaagctgcccgtgccctggcccaccctcgtgaccaccctgacctacggcgtgcagtgcttcagccgctaccccgacc
acatgaagcagcacgacttcttcaagtccgccatgcccgaaggctacgtccaggagcgcaccatcttcttcaaggacgacggcaactacaagacccgc
gccgaggtgaagttcgagggcgacaccctggtgaaccgcatcgagctgaagggcatcgacttcaaggaggacggcaacatcctggggcacaagctg
gagtacaactacaacagccacaacgtctatatcatggccgacaagcagaagaacggcatcaaggtgaacttcaagatccgccacaacatcgaggacg
gcagcgtgcagctcgccgaccactaccagcagaacacccccatcggcgacggccccgtgctgctgcccgacaaccactacctgagcacccagtccg
ccctgagcaaagaccccaacgagaagcgcgatcacatggtcctgctggagttcgtgaccgccgccgggatcactctcggcatggacgagctgtacaa
gtaatagtaagctggagcctcggtggccatgcttcttgccccttgggcctccccccagcccctcctccccttcctgcacccgtacccccgtggtctttgaat
aaagtctgagtgggcggcaactagtAAAAAAAAAAAAAAAAAAAAAAAAAAAAAAAAAAAAAAA
AAAAAAAAAAAAAAAAAAAAAAAAAAAAAAAAAAAAAggaagagc
SEQ ID NO. 36: pVAX1 vector sequence 
gactcttcgcgatgtacgggccagatatacgcgttgacattgattattgactagttattaatagtaatcaattacggggtcattagttcatagcccatatatgga
gttccgcgttacataacttacggtaaatggcccgcctggctgaccgcccaacgacccccgcccattgacgtcaataatgacgtatgttcccatagtaacgc
caatagggactttccattgacgtcaatgggtggactatttacggtaaactgcccacttggcagtacatcaagtgtatcatatgccaagtacgccccctattga
cgtcaatgacggtaaatggcccgcctggcattatgcccagtacatgaccttatgggactttcctacttggcagtacatctacgtattagtcatcgctattacca
tggtgatgcggttttggcagtacatcaatgggcgtggatagcggtttgactcacggggatttccaagtctccaccccattgacgtcaatgggagtttgttttg
gcaccaaaatcaacgggactttccaaaatgtcgtaacaactccgccccattgacgcaaatgggcggtaggcgtgtacggtgggaggtctatataagcag
agctctctggctaactagagaacccactgcttactggcttatcgaaattaatacgactcactatagggagacccaagctggctagcgtttaaacttaagcttg
 
gtaccgagctcggatccactagtccagtgtggtggaattctgcagatatccagcacagtggcggccgctcgagtctagagggcccgtttaaacccgctg
atcagcctcgactgtgccttctagttgccagccatctgttgtttgcccctcccccgtgccttccttgaccctggaaggtgccactcccactgtcctttcctaata
aaatgaggaaattgcatcgcattgtctgagtaggtgtcattctattctggggggtggggtggggcaggacagcaagggggaggattgggaagacaata
gcaggcatgctggggatgcggtgggctctatggcttctactgggcggttttatggacagcaagcgaaccggaattgccagctggggcgccctctggtaa
ggttgggaagccctgcaaagtaaactggatggctttctcgccgccaaggatctgatggcgcaggggatcaagctctgatcaagagacaggatgaggat
cgtttcgcatgattgaacaagatggattgcacgcaggttctccggccgcttgggtggagaggctattcggctatgactgggcacaacagacaatcggctg
ctctgatgccgccgtgttccggctgtcagcgcaggggcgcccggttctttttgtcaagaccgacctgtccggtgccctgaatgaactgcaagacgaggca
gcgcggctatcgtggctggccacgacgggcgttccttgcgcagctgtgctcgacgttgtcactgaagcgggaagggactggctgctattgggcgaagt
gccggggcaggatctcctgtcatctcaccttgctcctgccgagaaagtatccatcatggctgatgcaatgcggcggctgcatacgcttgatccggctacct
gcccattcgaccaccaagcgaaacatcgcatcgagcgagcacgtactcggatggaagccggtcttgtcgatcaggatgatctggacgaagagcatcag
gggctcgcgccagccgaactgttcgccaggctcaaggcgagcatgcccgacggcgaggatctcgtcgtgacccatggcgatgcctgcttgccgaatat
catggtggaaaatggccgcttttctggattcatcgactgtggccggctgggtgtggcggaccgctatcaggacatagcgttggctacccgtgatattgctg
aagagcttggcggcgaatgggctgaccgcttcctcgtgctttacggtatcgccgctcccgattcgcagcgcatcgccttctatcgccttcttgacgagttctt
ctgaattattaacgcttacaatttcctgatgcggtattttctccttacgcatctgtgcggtatttcacaccgcatacaggtggcacttttcggggaaatgtgcgc
ggaacccctatttgtttatttttctaaatacattcaaatatgtatccgctcatgagacaataaccctgataaatgcttcaataatagcacgtgctaaaacttcatttt
taatttaaaaggatctaggtgaagatcctttttgataatctcatgaccaaaatcccttaacgtgagttttcgttccactgagcgtcagaccccgtagaaaagat
caaaggatcttcttgagatcctttttttctgcgcgtaatctgctgcttgcaaacaaaaaaaccaccgctaccagcggtggtttgtttgccggatcaagagctac
caactctttttccgaaggtaactggcttcagcagagcgcagataccaaatactgtccttctagtgtagccgtagttaggccaccacttcaagaactctgtagc
accgcctacatacctcgctctgctaatcctgttaccagtggctgctgccagtggcgataagtcgtgtcttaccgggttggactcaagacgatagttaccgga
taaggcgcagcggtcgggctgaacggggggttcgtgcacacagcccagcttggagcgaacgacctacaccgaactgagatacctacagcgtgagct
atgagaaagcgccacgcttcccgaagggagaaaggcggacaggtatccggtaagcggcagggtcggaacaggagagcgcacgagggagcttcca
gggggaaacgcctggtatctttatagtcctgtcgggtttcgccacctctgacttgagcgtcgatttttgtgatgctcgtcaggggggcggagcctatggaaa
aacgccagcaacgcggcctttttacggttcctgggcttttgctggccttttgctcacatgttctt
SEQ ID NO. 41: *RecQ* 
GTGGCGCAGGCGGAAGTGTTGAATCTGGAGTCCGGAGCTAAACAGGTTTTACAAGAAACCTT
TGGCTACCAACAGTTTCGCCCCGGCCAGGAAGAAATTATCGACACTGTGCTTTCCGGCCGCG
ATTGCCTCGTCGTCATGCCCACTGGTGGCGGAAAATCCCTTTGCTATCAAATCCCTGCCTTATT
GCTAAACGGCCTTACCGTGGTTGTTTCACCGCTGATTTCGTTGATGAAAGATCAGGTGGATCA
ACTGCAAGCCAACGGCGTGGCGGCGGCGTGCCTTAACTCGACGCAAACCCGCGAACAGCAA
CTTGAAGTGATGACAGGCTGCCGCACCGGGCAAATTCGTCTGCTTTATATCGCCCCGGAACGC
CTGATGCTGGATAACTTTCTTGAGCATCTGGCGCACTGGAATCCGGTGTTATTAGCCGTTGATG
AAGCGCACTGTATCTCCCAATGGGGCCACGATTTCCGCCCGGAATATGCCGCGCTCGGTCAGT
TGCGCCAGCGGTTCCCGACGCTGCCGTTTATGGCGCTGACCGCCACAGCCGACGACACCACG
CGCCAGGATATCGTGCGCCTGCTGGGGCTGAACGATCCGCTGATTCAAATCAGCAGTTTTGAC
CGTCCGAATATTCGCTACATGCTGATGGAGAAGTTCAAACCGCTCGATCAGTTGATGCGCTAC
GTGCAGGAACAGCGCGGTAAGTCAGGCATTATCTACTGCAACAGCCGCGCGAAAGTAGAAGA
CACCGCTGCGCGCCTGCAAAGCAAGGGAATTAGCGCGGCGGCCTATCATGCCGGGCTGGAAA
ATAATGTTCGCGCCGATGTGCAGGAAAAATTCCAGCGCGATGACCTGCAAATTGTGGTGGCGA
CGGTGGCGTTCGGCATGGGCATCAATAAACCAAACGTTCGCTTCGTGGTCCACTTTGATATTC
CGCGCAATATCGAATCCTATTATCAGGAAACCGGACGCGCCGGGCGTGATGGCCTGCCCGCGG
AAGCGATGCTGTTTTACGATCCGGCTGATATGGCGTGGCTGCGCCGTTGTCTGGAAGAGAAGC
CGCAGGGGCAGTTGCAGGATATCGAGCGCCACAAACTCAATGCGATGGGCGCGTTTGCCGAA
GCGCAAACTTGCCGTCGTCTGGTATTGCTGAACTATTTTGGCGAAGGGCGTCAGGAGCCGTGC
GGGAACTGCGATATCTGCCTCGATCCGCCGAAACAGTACGACGGTTCAACCGATGCTCAGATT
GCCCTTTCCACCATTGGTCGTGTGAATCAGCGGTTTGGGATGGGTTATGTGGTGGAAGTGATT
CGTGGTGCTAATAACCAGCGTATCCGCGACTATGGTCATGACAAACTGAAAGTCTATGGCATG
GGCCGTGATAAAAGCCATGAACATTGGGTGAGCGTGATCCGCCAGCTGATTCACCTCGGCCTG
GTGACGCAAAATATTGCCCAGCATTCTGCCCTACAACTGACAGAGGCCGCGCGCCCGGTGCT
 
GCGCGGCGAATCCTCTTTGCAACTTGCCGTGCCGCGTATCGTGGCGCTCAAACCGAAAGCGA
TGCAGAAATCGTTCGGCGGCAACTATGATCGCAAACTGTTCGCCAAATTACGCAAACTGCGTA
AATCGATAGCCGATGAAAGTAATGTCCCGCCGTACGTGGTGTTTAACGACGCAACCTTGATTG
AGATGGCTGAACAGATGCCGATCACCGCCAGCGAAATGCTCAGCGTTAACGGCGTTGGGATG
CGCAAGCTGGAACGCTTTGGCAAACCGTTTATGGCGCTGATTCGTGCGCATGTTGATGGCGAT
GACGAAGAGTAG
SEQ ID NO. 42: lacIq promoter + lacI + rrnB T1 terminator + rrnB T2 terminator 
GACACCATCGAATGGTGCAAAACCTTTCGCGGTATGGCATGATAGCGCCCGGAAGAGAGTCA
ATTCAGGGTGGTGAATGTGAAACCAGTAACGTTATACGATGTCGCAGAGTATGCCGGTGTCTC
TTATCAGACCGTTTCCCGCGTGGTGAACCAGGCCAGCCACGTTTCTGCGAAAACGCGGGAAA
AAGTGGAAGCGGCGATGGCGGAGCTGAATTACATTCCCAACCGCGTGGCACAACAACTGGCG
GGCAAACAGTCGTTGCTGATTGGCGTTGCCACCTCCAGTCTGGCCCTGCACGCGCCGTCGCA
AATTGTCGCGGCGATTAAATCTCGCGCCGATCAACTGGGTGCCAGCGTGGTGGTGTCGATGGT
AGAACGAAGCGGCGTCGAAGCCTGTAAAGCGGCGGTGCACAATCTTCTCGCGCAACGCGTC
AGTGGGCTGATCATTAACTATCCGCTGGATGACCAGGATGCCATTGCTGTGGAAGCTGCCTGC
ACTAATGTTCCGGCGTTATTTCTTGATGTCTCTGACCAGACACCCATCAACAGTATTATTTTCTC
CCATGAAGACGGTACGCGACTGGGCGTGGAGCATCTGGTCGCATTGGGTCACCAGCAAATCG
CGCTGTTAGCGGGCCCATTAAGTTCTGTCTCGGCGCGTCTGCGTCTGGCTGGCTGGCATAAAT
ATCTCACTCGCAATCAAATTCAGCCGATAGCGGAACGGGAAGGCGACTGGAGTGCCATGTCC
GGTTTTCAACAAACCATGCAAATGCTGAATGAGGGCATCGTTCCCACTGCGATGCTGGTTGCC
AACGATCAGATGGCGCTGGGCGCAATGCGCGCCATTACCGAGTCCGGGCTGCGCGTTGGTGC
GGATATCTCGGTAGTGGGATACGACGATACCGAAGACAGCTCATGTTATATCCCGCCGTTAACC
ACCATCAAACAGGATTTTCGCCTGCTGGGGCAAACCAGCGTGGACCGCTTGCTGCAACTCTC
TCAGGGCCAGGCGGTGAAGGGCAATCAGCTGTTGCCCGTCTCACTGGTGAAAAGAAAAACC
ACCCTGGCGCCCAATACGCAAACCGCCTCTCCCCGCGCGTTGGCCGATTCATTAATGCAGCTG
GCACGACAGGTTTCCCGACTGGAAAGCGGGCAGTGAAATTCGCGCAACGCAATTAATGTAAG
TTAGCTCACTCATTAGGCACCAAATAAAACGAAAGGCTCAGTCGAAAGACTGGGCCTTTCGT
TTTATCTGTTGTTTGTCGGTGAACGCTCTCCTGAGTAGGACAAATCCGCCGGGAGCGGATTTG
AACGTTGCGAAGCAACGGCCCGGAGGGTGGCGGGCAGGACGCCCGCCATAAACTGCCAGGC
ATCAAATTAAGCAGAAGGCCATCCTGACGGATGGCCTTTT
SEQ ID NO. 43: *RecA* 
ATGGCTATCGACGAAAACAAACAGAAAGCGTTGGCGGCAGCACTGGGCCAGATTGAGAAAC
AATTTGGTAAAGGCTCCATCATGCGCCTGGGTGAAGACCGTTCCATGGATGTGGAAACCATCT
CTACCGGTTCGCTTTCACTGGATATCGCGCTTGGGGCAGGTGGTCTGCCGATGGGCCGTATCG
TCGAAATCTACGGACCGGAATCTTCCGGTAAAACCACGCTGACGCTGCAGGTGATCGCCGCA
GCGCAGCGTGAAGGTAAAACCTGTGCGTTTATCGATGCTGAACACGCGCTGGACCCAATCTA
CGCACGTAAACTGGGCGTCGATATCGACAACCTGCTGTGCTCCCAGCCGGACACCGGCGAGC
AGGCACTGGAAATCTGTGACGCCCTGGCGCGTTCTGGCGCAGTAGACGTTATCGTCGTTGACT
CCGTGGCGGCACTGACGCCGAAAGCGGAAATCGAAGGCGAAATCGGCGACTCTCACATGGG
CCTTGCGGCACGTATGATGAGCCAGGCGATGCGTAAGCTGGCGGGTAACCTGAAGCAGTCCA
ACACGCTGCTGATCTTCATCAACCAGATCCGTATGAAAATTGGTGTGATGTTCGGTAACCCGG
AAACCACTACCGGTGGTAACGCGCTGAAATTCTACGCCTCTGTTCGTCTCGACATCCGTCGTA
TCGGCGCGGTGAAAGAGGGCGAAAACGTGGTGGGTAGCGAAACCCGCGTGAAAGTGGTGA
AGAACAAAATCGCTGCGCCGTTTAAACAGGCTGAATTCCAGATCCTCTACGGCGAAGGTATCA
ACTTCTACGGCGAACTGGTTGACCTGGGCGTAAAAGAGAAGCTGATCGAGAAAGCAGGCGC
GTGGTACAGCTACAAAGGTGAGAAGATCGGTCAGGGTAAAGCGAATGCGACTGCCTGGCTGA
AAGATAACCCGGAAACCGCGAAAGAGATCGAGAAGAAAGTACGTGAGTTGCTGCTGAGCAA

CCCGAACTCAACGCCGGATTTCTCTGTAGATGATAGCGAAGGCGTAGCAGAAACTAACGAAG
ATTTTTAA
SEQ ID NO. 44: edited *RecA-RecA1* coding sequence 
ATCTACAGAGAAATCCGGCGTTGAGTTCGGGTTGCTCAGCAGCAACTCACGTACTTTCTTCTC
GATCTCTTTCGCGGTTTCCGGGTTATCTTTCAGCCAGGCAGTCGCATTCGCTTTACCCTGACCG
ATCTTCTCACCTTTGTAGCTGTACCACGCGCCTGCTTTCTCGATCAGCTTCTCTTTTACGCCCA
GGTCAACCAGTTCGCCGTAGAAGTTGATACCTTCGCCGTAGAGGATCTGGAATTCAGCCTGTT
TAAACGGCGCAGCGATTTTGTTCTTCACCACTTTCACGCGGGTTTCGCTACCCACCACGTTTT
CGCCCTCTTTCACCGCGCCGATACGACGGATGTCGAGACGAACAGAGGCGTAGAATTTCAGC
GCGTTACCACCGGTAGTGGTTTCCGGGTTACCGAACATCACACCAATTTTCATACGGATCTGGT
TGATGAAGATCAGCAGCGTGTTGGACTGCTTCAGGTTACCCGCCAGCTTACGCATCGCCTGGC
TCATCATACGTGCCGCAAGaCCCATGTGAGAGTCGtCGATTTCGCCTTCGATTTCCGCTTTCGGC
GTCAGTGCCGCCACGGAGTCAACGACGATAACGTCTACTGCGCCAGAACGCGCCAGGGCGTC
ACAGATTTCCAGTGCCTGCTCGCCGGTGTCCGGCTGGGAGCACAGCAGGTTGTCGATATCGA
CGCCCAGTTTACGTGCGTAGATTGGGTCCAGCGCGTGTTCAGCATCGATAAACGCACAGGTTT
TACCTTCACGCTGCGCTGCGGCGATCACCTGCAGCGTCAGCGTGGTTTTACCGGAAGATTCCG
GTCCGTAGATTTCGACGATACGGCCCATCGGCAGACCACCTGCCCCAAGCGCGATATCCAGTG
AAAGCGAACCGGTAGAGATGGTTTCCACATCCATGGAACGGTCTTCACCCAGGCGCATGATG
GAGCCTTTACCAAATTGTTTCTCAATCTGGCCCAGTGCTGCCGCCAACGCTTTCTGTTTGTTTT
CGTCGATAGCCAT
SEQ ID NO. 45: sgRNA targeting *RecQ*
   atggggccacgatttccgcc
SEQ ID NO. 46: sgRNA targeting *yghX*
   gggctggcctgcttacgagg
SEQ ID NO. 47: sgRNA targeting *RecA*
   ggctcatcatacgtgccgca
SEQ ID NO. 48: Donor sequence for *RecQ* knockout 
AGTTAGGCTTAAGTTACCCGATCACCAAACATGTGCGCCTTTATACTCAGGTTTACAGCGGCTA
TGGCGAATCGCTCATCGACTATAACTTCAACCAGACCCGTGTCGGTGTGGGGGTTATGCTAAA
CGATTTGTTTTGATGAACGGTTGAGTGGTTGGCAAATCTGGAATCCAGCATCCAGGATTACCC
TCTCAGAGACTAAAAGCATTGCAGTTTCTCGCGCAGGCGCTGAAAATAGCGCCTGTTTTTATT
TCAGGCAATCGGGGTGAATGTGGCGCAGGCGGAAGTGTTGAATCTGGAGTCCGGAGCTAAAC
AGGTTTTACAAGAAACCTTTGGCTACCAACAGTTTCGCCCCGGCCAGGAAGAAATTATCGAC
ACTGTGCTTTCCGGCCGCGATTGCCTCGTCGTCATGCCCACTGGTGGCGGAAAATCCCTTTGC
TATCAAATCCCTGCCTTATTGCTAAACGGCCTTACCGTGGTTGTTTCACCGCTGATTTCGCTGTT
CGCCAAATTACGCAAACTGCGTAAATCGATAGCCGATGAAAGTAATGTCCCGCCGTACGTGGT
GTTTAACGACGCAACCTTGATTGAGATGGCTGAACAGATGCCGATCACCGCCAGCGAAATGC
TCAGCGTTAACGGCGTTGGGATGCGCAAGCTGGAACGCTTTGGCAAACCGTTTATGGCGCTG
ATTCGTGCGCATGTTGATGGCGATGACGAAGAGTAGTCAGCAGCATAAAAAAGTGCCAGTATG
AAGACTCCGTAAACGTTTCCCCCGCGAGTCAAATGTATGTTGATGTTATTTCTCACCGTCGCCA
TGGTGCACATTGTGGCGCTTATGAGCCCCGGTCCCGATTTCTTTTTTGTCTCTCAGACCGCTGT
CAGTCGTTCCCGTAAAGAAGCGATGATGGGCGTGCTGGGCATTACCTGCGGCGTAATGGTTTG
GGCTGGGATTGCGCTGCTTGGCCTGCATTTGATTATCGAAAAAATGGCCTGGCT
SEQ ID NO. 49: Donor sequence for lacIq promoter + lacI + rrnB T1 terminator + rrnB T2 terminator knock-in 
TGGTTATCGATATCATGATACTGCGGCGGGGCGCTAAAACGAAGGGAAGAAGCATTAGTGTAA
TTAATTAAAGCAAGATAATACGTATGTTTGATCAATTTTCGAGGCGATAACCGGCCATAAAAAA
GCGCAACGTAGAACAGGAATTATTCTCACGAAAGAAATCCATTACCATCTCTTTATCCAGTCCA
TAGCGGCGCGTCAGAATACCCGCTTCCCAGGCGCTAAGCTGGCGATCTCCGGTCTTTTCGTAC
ATCCGGTGTGAGTAGCCTAATACAAAACCGCGTTTATAATCGGCACAGAATTTTGTCACATTAA
CCGCACTGTCGGCATGCGTTGCTTTTAATCCCGCCATTAAGCCTTTACCAAAATGGTTATTCAT
CGTTCACCTCATTCGCTATATATTGTCATATATAGCGATATTTTAGGCAAAGAACGACTTTTGCG
CACGCTATTTTTTCATGCTTGTTTAAGACGTAATCCCGTAAGAGATAAACCTATTCCGACACCA
TCGAATGGTGCAAAACCTTTCGCGGTATGGCATGATAGCGCCCGGAAGAGAGTCAATTCAGG
GTGGTGAATGTGAAACCAGTAACGTTATACGATGTCGCAGAGTATGCCGGTGTCTCTTATCAG
ACCGTTTCCCGCGTGGTGAACCAGGCCAGCCACGTTTCTGCGAAAACGCGGGAAAAAGTGG
AAGCGGCGATGGCGGAGCTGAATTACATTCCCAACCGCGTGGCACAACAACTGGCGGGCAA
ACAGTCGTTGCTGATTGGCGTTGCCACCTCCAGTCTGGCCCTGCACGCGCCGTCGCAAATTGT
CGCGGCGATTAAATCTCGCGCCGATCAACTGGGTGCCAGCGTGGTGGTGTCGATGGTAGAAC
GAAGCGGCGTCGAAGCCTGTAAAGCGGCGGTGCACAATCTTCTCGCGCAACGCGTCAGTGG
GCTGATCATTAACTATCCGCTGGATGACCAGGATGCCATTGCTGTGGAAGCTGCCTGCACTAAT
GTTCCGGCGTTATTTCTTGATGTCTCTGACCAGACACCCATCAACAGTATTATTTTCTCCCATGA
AGACGGTACGCGACTGGGCGTGGAGCATCTGGTCGCATTGGGTCACCAGCAAATCGCGCTGT
TAGCGGGCCCATTAAGTTCTGTCTCGGCGCGTCTGCGTCTGGCTGGCTGGCATAAATATCTCAC
TCGCAATCAAATTCAGCCGATAGCGGAACGGGAAGGCGACTGGAGTGCCATGTCCGGTTTTC
AACAAACCATGCAAATGCTGAATGAGGGCATCGTTCCCACTGCGATGCTGGTTGCCAACGATC
AGATGGCGCTGGGCGCAATGCGCGCCATTACCGAGTCCGGGCTGCGCGTTGGTGCGGATATCT
CGGTAGTGGGATACGACGATACCGAAGACAGCTCATGTTATATCCCGCCGTTAACCACCATCA
AACAGGATTTTCGCCTGCTGGGGCAAACCAGCGTGGACCGCTTGCTGCAACTCTCTCAGGGC
CAGGCGGTGAAGGGCAATCAGCTGTTGCCCGTCTCACTGGTGAAAAGAAAAACCACCCTGG
CGCCCAATACGCAAACCGCCTCTCCCCGCGCGTTGGCCGATTCATTAATGCAGCTGGCACGAC
AGGTTTCCCGACTGGAAAGCGGGCAGTGAAATTCGCGCAACGCAATTAATGTAAGTTAGCTC
ACTCATTAGGCACCAAATAAAACGAAAGGCTCAGTCGAAAGACTGGGCCTTTCGTTTTATCTG
TTGTTTGTCGGTGAACGCTCTCCTGAGTAGGACAAATCCGCCGGGAGCGGATTTGAACGTTG
CGAAGCAACGGCCCGGAGGGTGGCGGGCAGGACGCCCGCCATAAACTGCCAGGCATCAAAT
TAAGCAGAAGGCCATCCTGACGGATGGCCTTTTAGTAATCCAGCAACTCTTGTGGGAAATCTT
TGGCGGTTAAACGCGGCATCGTTGCACTCCTCAGTTGGTGTTTTTTTAAGCAAAGCATAAGCA
CGTATTTTTGCCCAGTTTTTCGTCACTCTGTGAGCCAGACTACGGGATACGCGCTGGCGAATC
GCTAAACTAGAAACATTGTTTCGAAATTGAACGGTGGAAAGGAGAGGTCATGGTCTGGTTAG
CGAATCCCGAACGTTACGGGCAGATGCAATACCGCTATTGCGGAAAAAGTGGTTTACGCCTGC
CCGCGTTATCGCTCGGTTTATGGCACAATTTCGGTCACGTTAACGCGCTGGAATCACAGCGTG
CGATCCTGCGTAAAGCGTTTGATTTGGGCATTACGCACTTTGATTTAGCCAACAATTACGGGCC
GCCTCCAGGAAGCGCAGAAGAGAACTTTGGTCGCCTGCTGCGGGAGGATTTTGCCGCTTATC
GCGATGAACTGATTATCTCTACCAAGGCTG
SEQ ID NO. 50: Donor sequence for mutating *RecA* to *RecA1*

ATCTACAGAGAAATCCGGCGTTGAGTTCGGGTTGCTCAGCAGCAACTCACGTACTTTCTTCTC
GATCTCTTTCGCGGTTTCCGGGTTATCTTTCAGCCAGGCAGTCGCATTCGCTTTACCCTGACCG
ATCTTCTCACCTTTGTAGCTGTACCACGCGCCTGCTTTCTCGATCAGCTTCTCTTTTACGCCCA
GGTCAACCAGTTCGCCGTAGAAGTTGATACCTTCGCCGTAGAGGATCTGGAATTCAGCCTGTT
TAAACGGCGCAGCGATTTTGTTCTTCACCACTTTCACGCGGGTTTCGCTACCCACCACGTTTT
CGCCCTCTTTCACCGCGCCGATACGACGGATGTCGAGACGAACAGAGGCGTAGAATTTCAGC

GCGTTACCACCGGTAGTGGTTTCCGGGTTACCGAACATCACACCAATTTTCATACGGATCTGGT
TGATGAAGATCAGCAGCGTGTTGGACTGCTTCAGGTTACCCGCCAGCTTACGCATCGCCTGGC
TCATCATACGTGCCGCAAGaCCCATGTGAGAGTCGtCGATTTCGCCTTCGATTTCCGCTTTCGGC
GTCAGTGCCGCCACGGAGTCAACGACGATAACGTCTACTGCGCCAGAACGCGCCAGGGCGTC
ACAGATTTCCAGTGCCTGCTCGCCGGTGTCCGGCTGGGAGCACAGCAGGTTGTCGATATCGA
CGCCCAGTTTACGTGCGTAGATTGGGTCCAGCGCGTGTTCAGCATCGATAAACGCACAGGTTT
TACCTTCACGCTGCGCTGCGGCGATCACCTGCAGCGTCAGCGTGGTTTTACCGGAAGATTCCG
GTCCGTAGATTTCGACGATACGGCCCATCGGCAGACCACCTGCCCCAAGCGCGATATCCAGTG
AAAGCGAACCGGTAGAGATGGTTTCCACATCCATGGAACGGTCTTCACCCAGGCGCATGATG
GAGCCTTTACCAAATTGTTTCTCAATCTGGCCCAGTGCTGCCGCCAACGCTTTCTGTTTGTTTT
CGTCGATAGCCATTTTTACTCCTGTCATGCCGGGTAATACCGGATAGTCAATATGTTCTGTTGAA
GCAAT

Although the present application has been described in conjunction with one or more embodiments, it should be understood that the present application is not limited to these embodiments. The descriptions in the present application are intended to encompass all variations and equivalents, which are all included within the spirit and scope of the appended claims. All documents cited herein are incorporated herein by reference in their entireties.

## Claims

**1.** The use of *Escherichia coli* host cells for the replication or expression of a nucleic acid molecule containing a poly(A) sequence, wherein the *Escherichia coli* host cell comprises a down-regulated gyrase activity or a down-regulated gyrase expression level.

**2.** The use according to claim 1, **characterized in that** the *Escherichia coli* host cell comprises the down-regulated gyrase activity due to a mutation in a *gyrA* gene or a *gyrB* gene.

**3.** The use according to claim 2, **characterized in that** the *Escherichia coli* host cell expresses a GyrA mutant due to the mutation in the *gyrA* gene, the GyrA mutant comprises:
i) an H80A mutation at position 80 corresponding to SEQ ID NO. 37, or
ii) A569T and T586A mutations at positions 569 and 586 corresponding to SEQ ID NO. 37.

**4.** The use according to claim 2, **characterized in that** the *Escherichia coli* host cell expresses a GyrB mutant due to the mutation in the *gyrB* gene, the GyrB mutant comprises:
i) an R136C mutation at position 136 corresponding to SEQ ID NO. 39,
ii) an E42D mutation at position 42 corresponding to SEQ ID NO. 39, or
iii) a D498A mutation at position 498 corresponding to SEQ ID NO. 39.

**5.** The use according to claim 2, **characterized in that** the *Escherichia coli* host cell expresses a GyrA mutant and a GyrB mutant due to mutations in the *gyrA* gene and the *gyrB* gene, wherein the GyrA mutant comprises an H80A mutation at position 80 corresponding to SEQ ID NO. 37, and the GyrB mutant comprises an R136C mutation at position 136 corresponding to SEQ ID NO. 39.

**6.** The use according to any one of claims 1-5, **characterized in that** the *Escherichia coli* host cell further comprises one or more of the following mutations:
i) a mutation in a *RecA* gene,
ii) an insertion of a *lacI* gene expression cassette, and
iii) a knockout of a *RecQ* gene.

**7.** The use according to claim 6, **characterized in that** the *Escherichia coli* host cell comprises:
i) the mutated *RecA* gene comprising a nucleotide sequence as set forth in SEQ ID NO. 44,
ii) the inserted *lacI* gene expression cassette comprising a nucleotide sequence as set forth in SEQ ID NO. 42, or
iii) the *RecQ* gene comprising a nucleotide sequence as set forth in SEQ ID NO. 41.

**8.** The use according to any one of claims 1-7, **characterized in that** the *Escherichia coli* host cell is JM108, NEB Stable, Top10, DH5α, DH10B, MG1655, AG1, BL21, DB3.1, DC10B, DH1, E.Cloni 10G, EPI300, EPI400, JM109, JM110, STBL2, STBL3, SURE, TOP10F', XL1-Blue or XL10-Gold that has been engineered to comprise the down-regulated gyrase activity or the down-regulated gyrase expression level.

**9.** The use according to any one of claims 1-8, **characterized in that** the *Escherichia coli* host cell comprises a vector comprising the nucleic acid molecule containing the poly(A) sequence, or
the *Escherichia coli* host cell comprises the nucleic acid molecule containing the poly(A) sequence in the genome thereof.

**10.** The use according to any one of claims 1-9, **characterized in that** the poly(A) sequence in the nucleic acid molecule containing the poly(A) sequence comprises:
i) 20-250 consecutive A bases; or
ii) 2-5 consecutive A base stretches separated by a non-A base, wherein each consecutive A base stretch comprises 10-100 consecutive A bases that are separated from each other by 1-20 non-A bases.

**11.** A recombinant *Escherichia coli host cell*, **characterized in that** the recombinant *Escherichia coli* host cell is JM108, NEB Stable, Top10, DH5α, DH10B, MG1655, AG1, BL21, DB3.1, DC10B, DH1, E.Cloni 10G, EPI300, EPI400, JM109, JM110, STBL2, STBL3, SURE, TOP10F', XL1-Blue or XL10-Gold that has been engineered to comprise a down-regulated gyrase activity or a down-regulated gyrase expression level.

**12.** A recombinant *Escherichia coli* host cell, **characterized in that** the recombinant *Escherichia coli* host cell comprises a down-regulated gyrase activity due to a mutation in a *gyrA* gene or a *gyrB* gene.

**13.** The recombinant *Escherichia coli* host cell according to claim 11 or 12, **characterized in that** the recombinant *Escherichia coli* host cell expresses a GyrA mutant comprising:
i) an H80A mutation at position 80 corresponding to SEQ ID NO. 37, or
ii) A569T and T586A mutations at positions 569 and 586 corresponding to SEQ ID NO. 37.

**14.** The recombinant *Escherichia coli* host cell according to claim 11 or 12, **characterized in that** the recombinant *Escherichia coli* host cell expresses a GyrB mutant comprising:
i) an R136C mutation at position 136 corresponding to SEQ ID NO. 39,
ii) an E42D mutation at position 42 corresponding to SEQ ID NO. 39, or
iii) a D498A mutation at position 498 corresponding to SEQ ID NO. 39.

**15.** The recombinant *Escherichia coli* host cell according to claim 11 or 12, **characterized in that** the *Escherichia coli* host cell expresses the GyrA mutant and the GyrB mutant due to mutations in the *gyrA* gene and the *gyrB* gene, wherein the GyrA mutant comprises an H80A mutation at position 80 corresponding to SEQ ID NO. 37, and the GyrB mutant comprises an R136C mutation at position 136 corresponding to SEQ ID NO. 39.

**16.** The recombinant *Escherichia coli* host cell according to any one of claims 11-15, **characterized in that** the recombinant *Escherichia coli* host cell further comprises one or more of the following mutations:
i) a mutation in a *RecA* gene,
ii) an insertion of a *lacI* gene expression cassette, and
iii) a knockout of a *RecQ* gene.

**17.** The recombinant *Escherichia coli* host cell according to any one of claims 11-15, **characterized in that** the recombinant *Escherichia coli* host cell further comprises:
i) a mutation in a *RecA* gene, wherein the mutated *RecA* gene comprising a nucleotide sequence as set forth in SEQ ID NO. 44,
ii) an insertion of a *lacI* gene expression cassette, wherein the inserted *lacIq* gene expression cassette comprising a nucleotide sequence as set forth in SEQ ID NO. 42, or
iii) a knockout of a *RecQ* gene, wherein the *RecQ* gene comprising a nucleotide sequence as set forth in SEQ ID NO. 41.

**18.** The recombinant *Escherichia coli* host cell according to claim 16 or 17, **characterized in that** the recombinant *Escherichia coli* host cell comprises:
i) the mutation in the *gyrA* gene, wherein the mutated *gyrA* gene comprising a nucleotide sequence as set forth in SEQ ID NO: 3 or 4,
ii) the mutation in the *RecA* gene, wherein the mutated *RecA* gene comprising a nucleotide sequence as set forth in SEQ ID NO. 44,
iii) the insertion of the *lacI* gene expression cassette, wherein the inserted *lacIq* gene comprising a nucleotide sequence as set forth in SEQ ID NO. 42, and
iv) the knockout of the *RecQ* gene, wherein the *RecQ* gene comprising a nucleotide sequence as set forth in SEQ ID NO. 41.

**19.** A method for replication of a nucleic acid molecule containing a poly(A) sequence using an *Escherichia coli* host cell, **characterized in that**
the method comprises:
i) introducing a vector comprising the nucleic acid molecule containing the poly(A) sequence into the *Escherichia coli* host cell according to any one of claims 11 to 18, and
ii) culturing the *Escherichia coli* host cell under a condition that facilitates replication of the vector.
19. A method for expression of a nucleic acid molecule containing a poly(A) sequence using an *Escherichia coli* host cell, **characterized in that**
the method comprises:
i) introducing a vector comprising the nucleic acid molecule containing the poly(A) sequence into the *Escherichia coli* host cell according to any one of claims 11 to 18,
ii) optionally, culturing the *Escherichia coli* host cell under a condition that facilitates replication of the vector, and
iii) culturing the *Escherichia coli* host cell under a condition that facilitates expression of the vector, or
extracting the vector from the *Escherichia coli* host cell of step i) or ii) and performing in vitro transcription on the vector; alternatively
i) integrating the nucleic acid molecule containing the poly(A) sequence into the genome of the *Escherichia coli* host cell according to any one of claims 11-18, and
ii) culturing the *Escherichia coli* host cell under a condition that facilitates expression of the nucleic acid molecule containing the poly(A) sequence.

**20.** A method for preparing a recombinant *Escherichia coli* host cell, **characterized in that** the method comprises:
i) providing an *Escherichia coli* host cell comprising a *gyrA* gene and a *gyrB* gene,
ii) down-regulating a gyrase activity in the *Escherichia coli* host cell, or down-regulating a gyrase expression level in the *Escherichia coli* host cell.

**21.** The method according to claim 20, **characterized in that** step ii) comprises,
1) engineering the *gyrA* gene to comprise a nucleotide sequence as set forth in SEQ ID NO. 3,
2) engineering the *gyrB* gene to comprise a nucleotide sequence as set forth in SEQ ID NO. 6,
3) engineering the *gyrA* gene to comprise a nucleotide sequence as set forth in SEQ ID NO. 4,
4) engineering the *gyrB* gene *to* comprise a nucleotide sequence as set forth in SEQ ID NO. 5, or
5) engineering the *gyrB* gene to comprise a nucleotide sequence as set forth in SEQ ID NO. 7.

**22.** The method according to claim 21, **characterized in that** step ii) comprises introducing into the *Escherichia coli* host cell of step i) a Cas9 enzyme, and
1) an sgRNA comprising a nucleotide sequence as set forth in SEQ ID NO. 8 and a donor sequence comprising a nucleotide sequence as set forth in SEQ ID NO. 13,
2) an sgRNA comprising a nucleotide sequence as set forth in SEQ ID NO. 11 and a donor sequence comprising a nucleotide sequence as set forth in SEQ ID NO. 16,
3) an sgRNA comprising a nucleotide sequence as set forth in SEQ ID NO. 9 and a donor sequence comprising a nucleotide sequence as set forth in SEQ ID NO. 14,
4) an sgRNA comprising a nucleotide sequence as set forth in SEQ ID NO. 10 and a donor sequence comprising a nucleotide sequence as set forth in SEQ ID NO. 15, or
5) an sgRNA comprising a nucleotide sequence as set forth in SEQ ID NO. 12 and a donor sequence comprising a nucleotide sequence as set forth in SEQ ID NO. 17.

**23.** The method according to any one of claims 20-22, **characterized in that** the *Escherichia coli* host cell further comprises any one or more of the following operation steps:
1) mutating a *RecA* gene,
2) inserting a *lacI* gene expression cassette, and
3) knocking out a *RecQ* gene.

**24.** The method according to any one of claims 20-23, **characterized in that** the *Escherichia coli* host cell in step i) is selected from JM108, NEB Stable, Top10, DH5α, DH10B, MG1655, AG1, BL21, DB3.1, DC10B, DH1, E.Cloni 10G, EPI300, EPI400, JM109, JM110, STBL2, STBL3, SURE, TOP10F', XL1-Blue or XL10-Gold.
